(19) **Europäisches Patentamt / European Patent Office / Office européen des brevets**

(11) **EP 1 996 717 B1**

(12) **EUROPEAN PATENT SPECIFICATION**

(45) Date of publication and mention of the grant of the patent:
**12.11.2014 Bulletin 2014/46**

(51) Int Cl.:
*G01N 33/543* (2006.01)   *G01N 33/50* (2006.01)
*G01N 33/53* (2006.01)

(21) Application number: **07751131.9**

(22) Date of filing: **20.02.2007**

(86) International application number:
**PCT/US2007/004349**

(87) International publication number:
**WO 2007/098148 (30.08.2007 Gazette 2007/35)**

(54) **METHODS AND ARRAYS FOR TARGET ANALYTE DETECTION AND DETERMINATION OF TARGET ANALYTE CONCENTRATION IN SOLUTION**

VERFAHREN UND ANORDNUNGEN ZUR DETEKTION VON ZIELANALYTEN UND BESTIMMUNG DER ZIELANALYTENKONZENTRATION IN EINER LÖSUNG

PROCÉDÉS ET RÉSEAUX POUR LA DÉTECTION D'ANALYTES CIBLES ET LA DÉTERMINATION DE LA CONCENTRATION D'ANALYTES CIBLES EN SOLUTION

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.02.2006 US 775692 P**
**17.04.2006 US 792736 P**

(43) Date of publication of application:
**03.12.2008 Bulletin 2008/49**

(60) Divisional application:
**12177276.8 / 2 538 220**

(73) Proprietor: **TRUSTEES OF TUFTS COLLEGE**
**Medford, MA 02155 (US)**

(72) Inventors:
• **WALT, David R.**
  **Boston, MA 02116 (US)**
• **RISSIN, David M.**
  **Medford, MA 02155 (US)**
• **GORRIS, Hans-Heiner**
  **Medford, MA 02155 (US)**

(74) Representative: **Hoffmann Eitle**
**Patent- und Rechtsanwälte PartmbB**
**Arabellastraße 30**
**81925 München (DE)**

(56) References cited:
**WO-A-99/58948    WO-A2-2007/044974**

**US-A- 6 001 564    US-A- 6 143 496**
**US-B2- 6 582 908**

• **RISSIN DAVID M ET AL: "Digital concentration readout of single enzyme molecules using femtoliter arrays and Poisson statistics." NANO LETTERS MAR 2006, vol. 6, no. 3, 21 February 2006 (2006-02-21), pages 520-523, XP002483266 ISSN: 1530-6984**
• **RISSIN DAVID M ET AL: "Digital readout of target binding with attomole detection limits via enzyme amplification in femtoliter arrays." JOURNAL OF THE AMERICAN CHEMICAL SOCIETY 17 MAY 2006, vol. 128, no. 19, 20 April 2006 (2006-04-20), pages 6286-6287, XP002483267 ISSN: 0002-7863**
• **RONDELEZ YANNICK ET AL: "Microfabricated arrays of femtoliter chambers allow single molecule enzymology" NATURE BIOTECHNOLOGY, NATURE PUBLISHING GROUP, NEW YORK, NY, US, vol. 23, no. 3, 1 March 2005 (2005-03-01), pages 361-365, XP002431619 ISSN: 1087-0156**
• **ANGENENDT PHILIPP ET AL: "Subnanoliter enzymatic assays on microarrays" PROTEOMICS, vol. 5, no. 2, February 2005 (2005-02), pages 420-425, XP002537616 ISSN: 1615-9853**
• **NIEMEYER ET AL.: 'Combination of DNA-directed immobilization and immuno-PCR: Very Sensitive Antigen Detection by Means of Self-Assembled DNA-Protein Conjugates' NUC. ACIDS RES. vol. 31, no. 16, 2003, XP008130337**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 996 717 B1

- **SYKES P J ET AL: "QUANTITATION OF TARGETS FOR PCR BY USE OF LIMITING DILUTION", BIOTECHNIQUES, vol. 13, no. 3, 1992, pages 444-449, ISSN: 0736-6205**

**Description**

**Government Rights**

The United States government may have certain rights in this invention pursuant to Contract No. N00014-01-1 awarded by the Department of Defense, Defense Advanced Research Projects Agency (DARPA) Office of Naval Research.

**Background**

Methods that implement high-sensitivity and low-level analyte detection in conjunction with rapid and reproducible experimental protocols are the cornerstone of modem analytical measurements. Currently, most known techniques for quantifying low levels of target analyte in a sample matrix use amplification procedures to increase the number of reporter molecules and thereby provide a measurable signal. These known processes include enzyme-linked immunosorbent assays (ELISA) for amplifying the signal in antibody-based assays, as well as the polymerase chain reaction (PCR) for amplifying target DNA strands in DNA-based assays. A more sensitive but indirect protein target amplification technique, called immuno-PCR (*see* Sano, T.; Smith, C. L.; Cantor, C. R. Science 1992, 258, 120-122), makes use of oligonucleotide markers, which can subsequently be amplified using PCR and detected using a DNA assay (*see* Nam, J. M.; Thaxton, C. S.; Mirkin, C. A. Science 2003, 301, 1884-1886; Niemeyer, C. M.; Adler, M.; Pignataro, B.; Lenhert, S.; Gao, S.; Chi, L. F.; Fuchs, H.; Blohm, D. Nucleic Acids Research 1999, 27, 4553-4561; and Zhou, H.; Fisher, R. J.; Papas, T. S. Nucleic Acids Research 1993, 21, 6038-6039). While the immuno-PCR method permits ultra low-level protein detection, it is a complex assay procedure, and can be prone to false-positive signal generation (*see* Niemeyer, C. M.; Adler, M.; Wacker, R. Trends in Biotechnology 2005, 23, 208-216).

One disadvantage of these known methods is their reliance on separate steps to amplify reporter molecules to provide a measurable signal, thereby requiring additional amplification steps and thus additional time, equipment, and materials.

In addition, known methods for accurately quantifying the concentration of a particular analyte in solution are all based on ensemble responses in which many analyte molecules give rise to the measured signal.

For example, WO 99/58948 A describes an optically-based method and system for analyte detection using solid phase immobilization, specific analyte labels adapted for signal generation and corresponding processes for the utilization thereof. The system is comprised of a platform/support for immobilizing a sample stage having a labeled sample (analyte complex) bound thereto, a radiation source, an optical apparatus for generating and directing radiation at said sample and a means for data collection and analysis.

Rondelez et al. (see bibliography) describe a silicone device with a large array of micrometer-sized cavities which can be used to tightly enclose femtolitres of solution over long periods of time. The device described therein could reportedly measure the activity of single molecules of β-galactosidase and horseradish peroxidise.

Angenendt et al. (see bibliography) describe multiplex enzymatic assays in subnanolitre volumes on a single microarray using multiple spotting technology and reported a measurement sensitivity down to 35 enzyme molecules. The assay was applied to the detection of a prognostic marker, cathepsin.

WO 2007/044974 A2 describes methods and devices for absolute quantification of polymerase chain reaction target nucleic acids. In particular, the methods and devices provide for splitting a nucleic acid sample to be analyzed into small, isolated volumes, conducting the method of polymerase chain reaction (PCR) on said volumes, then detecting and analyzing the PCR amplification products before performing absolute quantification.

Therefore, there is a need in the art for an improved method and system of target analyte detection.

**Brief Summary of the Invention**

The present invention, as defined by the claims, is a method of measuring the concentration of a target analyte in a sample. The method comprises:

(a) providing a sample and an array of capture components, said sample comprising molecules of target analyte, said array comprising at least 1000 sites, each site having a defined volume between 10 attoliters and 50 picoliters;
(b) contacting said array with said sample so that sites contain target analyte bound to capture components such that the ratio of molecules of target analyte to sites of said array is less than 1:5 which results in sites with single analyte molecules; and
(c) determining the percentage of sites of said array which contain a target analyte molecule to provide a measure of the target analyte concentration.

The present disclosure outlines exemplary methods, systems and devices that can be used to perform the method of the invention.

According to one embodiment, the present disclosure relates to a method of detecting a target analyte in a sample. The method includes providing an array comprising a plurality of sites, each site comprising a capture component, and contacting the array with the sample such that each site in a subset of the plurality of sites contains a single target analyte. Each target analyte comprises an enzymatic component. The method further includes contacting the array with an enzymatic substrate and detecting a change in an optical property at each of the sites as an indication of the presence of the target analyte.

The present disclosure, in another embodiment, relates to a method of detecting target analytes in a sample. The method includes providing an array comprising a plurality of sites, and contacting the array with the sample such that each site in a first subset of the plurality of sites contains a single first target analyte and each site in a second subset of the plurality of sites contains a single second target analyte. In this embodiment, each site comprises a capture component and each of the first and second target analytes comprises an enzymatic component. The method further includes contacting the array with a first enzymatic substrate and detecting any change in an optical property as a result of the first enzymatic substrate at each of the sites as an indication of the presence of one of the first or second target analytes. In addition, the method includes washing the array and contacting the array with a second enzymatic substrate. Further, the method includes detecting any change in an optical property as a result of the second enzymatic substrate at each of the sites as an indication of the presence of one of the first or second target analytes.

In accordance with another embodiment, the present disclosure relates to a method of detecting a target analyte in a sample. The method includes providing an array comprising a plurality of sites and contacting the array with the sample such that each site in a subset of the plurality of sites contains a single target analyte. In this method, each site comprises a capture component. The method also includes contacting each of the single target analytes with a binding ligand comprising an enzymatic component and further contacting the array with an enzymatic substrate. In addition, the method includes detecting a change in an optical property at each of the sites as an indication of the presence of the target analyte.

The present disclosure, according to a further embodiment, is a method of quantifying an amount of a target analyte in a sample. The method includes providing an array comprising a plurality of sites, each site comprising a capture component and contacting the array with the sample such that each site in a subset of the plurality of sites contains a single target analyte. In this embodiment, each target analyte comprises an enzymatic component. The method also includes contacting the array with an enzymatic substrate, detecting a change in an optical property at each of the sites as an indication of the presence of the target analyte, and calculating an amount of the target analyte in the sample.

In one embodiment, the present disclosure contemplates a method of producing an array of single molecules, comprising: providing a sample and an array, said sample comprising a molecules of target analyte in a concentration, said array comprising at least 1000 sites, each site having a defined volume between 10 attoliters and 50 picoliters; and contacting said array with said sample such that the ratio of molecules of target analyte to each site of said array is less than 1:1, so as to produce an array comprising sites containing single molecules. In one embodiment, the present disclosure contemplates the array, as a composition of matter, produced according to this method. It is not intended that the present disclosure be limited by the nature of the target analyte. In one embodiment said target analyte comprises biomolecules (for example, proteins, nucleic acids, lipids, and carbohydrates; alternatively, hormones, cytokines and cellular antigens; preferably, receptors such as neural receptors, hormonal receptors, and nutrient receptors). It is not intended that the present disclosure be limited by the nature of the biomolecule. In one embodiment, said biomolecule is selected from the group consisting of proteins, nucleic acids, lipids, and carbohydrates. In another embodiment, said biomolecule is selected from the group consisting of hormones, cytokines and cellular antigens. In yet another embodiment, said biomolecule is a receptor (e.g. neural receptors, hormonal receptors, and nutrient receptors). In one embodiment, said target analyte is an environmental pollutant (e.g. pesticides, insecticides and toxins). In one embodiment, said target analyte is a drug. When used in a method according to the present invention, the ratio is chosen so that one achieves sites with single molecules. For the invention, the ratio is chosen so that the readout will be a digital readout (e.g. each site will have either one molecule or none); that is, the ratio is less than 1:5. In another embodiment, said ratio is 1:10. In still another embodiment, said ratio is between 1:5 and 1:500. In one embodiment, the percentage of sites containing a single molecule provide a measure of the concentration of molecules in said sample (in other words, by counting and knowing the number of total sites and the defined volume of the sites).

In one embodiment, the method further comprises, after step b), sealing the array such that the contents of each site cannot escape said site. In one embodiment, the present invention contemplates the sealing of molecules in solution, the capture of molecules in solution (e.g. with a capture component) before sealing, and/or the capture of molecules from the solution (and in some embodiments, the removal of molecules from the solution) before sealing. In one embodiment, the present disclosure contemplates the sealed array, produced according to the method.

It is not intended that the present disclosure be limited by the nature of the capture component. In one embodiment, wherein each site of said array comprises a capture component. In another embodiment, said target analyte comprises a single-stranded nucleic acid, and said capture component comprises a complementary nucleic acid

It is not intended that the present disclosure be limited to any particular volume. However, in one embodiment, said defined volume is measured in femtoliters. In one embodiment, the present disclosure contemplates, as a composition of matter, an array of sites, said sites of defined femtoliter volume.said defined volume is the same at each site and ranges from about 30 femtoliters to about 60 femtoliters. In one embodiment, said defined femtoliter volume is 46 femtoliters.

It is not intended that the present disclosure be limited to the number of sites. While 1000 sites may be used, preferably more sites are employed (e.g. more than 10,000, between 20,000 and 30,000 sites, and still more preferably between 100,000 and 10,000,000 sites.

The present invention contemplates a method of measuring the concentration of a target analyte in a sample, the method comprising: providing a sample and an array, said sample comprising a biomolecule target analyte, said array comprising at least 1000 sites, each site having a defined volume between 10 attoliters and 50 picoliters; contacting said array with said sample such that the ratio of biomolecule target analyte to each site of said array is less than 1:5; and determining the number of sites of said array which contain a single biomolecule. The ratio is chosen so that one achieves sites with single molecules. In a preferred, embodiment, the ratio is chosen so that the readout will be a digital readout (e.g. each site will have either one molecule or none). In another embodiment, said ratio is 1:10. In still another embodiment, said ratio is between 1:5 and 1:500. In one embodiment, the percentage of sites containing a single molecule provide a measure of the concentration of molecules in said sample (in other words, by counting and knowing the number of total sites and the defined volume of the sites).

Preferably, the biomolecules are labeled (e.g. with a fluorophore). In one embodiment, said determining comprises detecting a change in an optical property at said sites as an indication of the presence of said target analyte. Again, it is preferred that the percentage of sites containing a single biomolecule provides a measure of the concentration of biomolecules in said sample.

In one embodiment, the method further comprises, after the contacting step but before the determining step, sealing the array such that the contents of each site cannot escape said site. In one embodiment, the present invention contemplates the sealing of molecules in solution, the capture of molecules in solution (e.g. with a capture component) before sealing, and/or the capture of molecules from the solution (and in some embodiments, the removal of molecules from the solution) before sealing.

In yet another embodiment, the present invention contemplates a method of measuring the concentration of a target analyte in a sample, the method comprising: providing a sample and an array, said sample comprising a biomolecule target analyte in a first concentration, said array comprising at least 10,000 sites, each site having a defined femtoliter volume; diluting said sample to create a diluted sample, said diluted sample comprising a biomolecule target analyte in a second concentration; contacting said array with said diluted sample such that the ratio of biomolecule target analyte to each site of said array is between 1:5 and 1:500; and determining the number of sites of said array which contain a single biomolecule.

The present disclosure contemplates a method of producing an array of single cells, the method comprising: providing a sample and an array, said sample comprising cells, said array comprising at least 1000 sites, each site having a defined volume between 10 attoliters and 50 picoliters; and contacting said array with said sample such that the ratio of cells to each site of said array is less than 1:1, so as to produce an array comprising sites containing single molecules. In one embodiment, the present disclosure contemplates, as a composition of matter, the array produced according to this embodiment of the method. In one embodiment of the method, the present invention further contemplates the step of determining which sites of the array contain single cells. Preferably, the percentage of sites containing a single cell provide a measure of the concentration of cells in said sample. It is not intended that the present disclosure be limited by the nature of the cells. Prokaryotic cells are contemplates (e.g. bacterial cells) as well as eukaryotic cells (e.g. mammalian cells such as tumor cells and nerve cells. Although it is not necessary to understand the mechanism of an invention, it is believed that an array of single cells allows capture of a single mRNA target per well. In one embodiment, the single cells are trapped and subsequently lysed. In one embodiment, a single well comprises a single cell. In one embodiment, the single cell within the single well is lysed. In one embodiment, the targets from the single cell are trapped within the single well. In another embodiment, a single cell is lysed and the mRNA targets are subsequently captured. For example, one single cell is lysed wherein a plurality of mRNA targets are captured in wells with no more than one copy of each transcript type per well. Although it is not necessary to understand the mechanism of an invention, it is believed that while a cell may comprise thousands of different transcripts with may copies of each per cell, the present invention contemplates capturing no more than one of each different transcript type per well (i.e., for example, each well comprises no more than one copy of each transcript). In one embodiment, the single cell comprises multiple targets (i.e., for example, mRNA types). In one embodiment, the multiple targets (i.e., for example, mRNA types) are sequentially interrogated thereby determining their presence (i.e., for example, by using different colored fluorophores). In one embodiment, the targets (i.e., for example, mRNA types) are quantitatively determined by detecting a digital signal.

In still another embodiment, the present disclosure contemplates a method of producing an array comprising a plurality of different cells, the method comprising: providing a sample and an array, said sample comprising cells, said array comprising at least 1000 sites, each site having a defined volume between 10 attoliters and 50 picoliters; and contacting said array with said sample. In one embodiment, the plurality of different cells are lysed, thereby forming a lysate comprising multiple targets. In one embodiment, each of the plurality of different cells are lysed within different wells. In one embodiment, the plurality of different cells are lysed within a single well. In one embodiment, the lysates from the plurality of different cells are pooled. In one embodiment, the pooled lysates are captured. In one embodiment, the multiple targets (i.e., for example, mRNA types) are sequentially interrogated thereby determining their presence (i.e., for example, by using different colored fluorophores). In one embodiment, the targets (i.e., for example, mRNA types) are quantitatively determined by detecting a digital signal.

In one embodiment, the method further comprises, after contacting the array with the cells, sealing the array such that the contents of each site cannot escape said site. In one embodiment, the present invention contemplates the sealing of cells in solution, the capture of cells in solution (e.g. with a capture component) before sealing, and/or the capture of cells from the solution (and in some embodiments, the removal of cells from the solution) before sealing. In one embodiment, the present disclosure contemplates the sealed array comprising cells, produced according to the above-described method. In one embodiment, cells are immobilized by said capture component (e.g. a receptor, antibody or protein, such as fibronectin) after contacting said array (e.g. the capture component is present, and preferably attached, at each site of the array).

Again, it is not intended that the present invention be limited by the ratio of cells to sites provided that the ratio is less than 1:5. In another embodiment, said ratio is 1:10. In still another embodiment, said ratio is between 1:5 and 1:500. In one embodiment, the percentage of sites containing a single cell provide a measure of the concentration of molecules in said sample (in other words, by counting and knowing the number of total sites and the defined volume of the sites).

In one embodiment, the present disclosure contemplates a method of detecting cells in a sample, the method comprising: providing a sample and an array, said sample comprising cells in a first concentration, said array comprising at least 1000 sites, each site having a defined volume between 10 attoliters and 50 picoliters; diluting said sample to create a diluted sample, said diluted sample comprising cells in a second concentration; contacting said array with said diluted sample such that the ratio of cells to each site of said array is less than 1:5; and determining the number of sites of said array which contain a single cell. This array is preferably sealed prior to said determining step and the present invention contemplates both the array and the sealed array as compositions of matter.

In still another embodiment, the present disclosure contemplates a method of detecting a target analyte of a cell in a sample, the method comprising: providing a sample and an array, said sample comprising cells in a first concentration, each cell comprising a target analyte, said array comprising at least 1000 sites, each site having a defined femtoliter volume; diluting said sample to create a diluted sample, said diluted sample comprising cells in a second concentration; contacting said array with said diluted sample such that the ratio of cells to each site of said array is less than 1:5; treating said cells in said array under conditions such that said cells are lysed; and determining the number of sites of said array which contain a target analyte of said cells. This array is preferably sealed prior to said determining step and the present disclosure contemplates, as a composition of matter, the array and the sealed array produced in this fashion.

In alternative embodiment, the present disclosure contemplates a method of detecting a target analyte of a cell in a sample, the method comprising: providing a sample and an array, said sample comprising cells in a concentration, said array comprising at least 1000 sites, each site having a defined femtoliter volume; treating said cells under conditions such that said cells are lysed so as to create a treated sample; contacting said array with said treated sample such that the ratio of target analyte to each site of said array is less than 1:5; and determining the number of sites of said array which contain a target analyte. Again, the array is preferably sealed prior to said determining step and the present disclosure contemplates, as a composition of matter, the array and the sealed array produced in this manner.

In one embodiment, the cells are prokaryotic cells. In a particular embodiment, the cells are bacterial cells and the target analyte is a bacterial enzyme released from the cells after lysis. In a preferred embodiment, each site contains a capture reagent directed at capturing the target analyte after lysis.

A method of detecting a reaction component that affects a reaction, the method comprising: providing a reaction component, a sample and an array, said sample comprising a target analyte in a first concentration, said array comprising at least 1000 sites, each site having a defined volume between 10 attoliters and 50 picoliters; contacting said array with said sample such that the ratio of target analyte to each site of said array is less than 1:5; introducing said reaction component to each site of said array, and determining whether the reaction component affects a reaction. In a preferred embodiment, said target analyte is an enzyme and substrate for said enzyme is present in each site of said array prior to the determining step. In a particularly preferred embodiment, said reaction component is an enzyme inhibitor and said determining step comprises determining whether said enzyme inhibitor inhibits the reaction of said enzyme with said substrate. In one embodiment, the present disclosure contemplates sealing the array prior to the determining step, and both the array and the seal array are contemplated as compositions of matter.

The present invention specifically contemplate embodiments where the rate constants of enzyme are measured in single enzyme molecule settings. For example, in embodiment, the enzyme and inhibitor are preincubated prior to introduction into the sites of the array (e.g. in an aqueous buffer system) together in bulk, such that the inhibitor concentration is higher than the dissociation constant for inhibitor binding ($K_i$) and all of the active sites of the enzyme are likely to be blocked or occupied. As $K_i$ differs for different enzyme/inhibitor pairs, the concentration of inhibitor must be adjusted accordingly. With some particular enzyme/inhibitor pairs exemplified herein, a variety of inhibitor starting concentrations can be employed, ranging between 100 mM and 20 $\mu$M. In a second step of this embodiment of the method, one or more substrates (e.g. a chromogenic substrate) in an aqueous buffer system are added, preferably in a high volume excess (between 10-fold and 100,000-fold). In a third step of this embodiment of the method, the bulk solution is added to the array of sites, each site having a defined volume (between 10 attoliters and 50 picoliters, more preferably femtoliters). At this point, the inhibitor is not in excess any more; indeed, the concentration of the inhibitor is much less than the $K_i$ (it is preferred that less than 100,000 molecules of inhibitor, and preferably less than 10,000 molecules of inhibitor, and still more preferably less than 3,000 molecules of inhibitor, and even more preferably less than 1,000 molecules of inhibitor, are remaining in the solution). At the point where the solution is added to the sites of the array, the enzyme concentration is such (due to the many fold dilution of step 2) there will be a single enzyme in some sites and no enzyme in others. In a preferred embodiment, the array is then monitored for signal over time with a detection means (e.g. so that the utilization of substrate by the enzyme can be detected). When the frequency distribution of substrate turnover onset times is plotted, the $K_{off}$ rate constant can be calculated.

In another embodiment of such a method, the concentration of the inhibitor is chosen such that it is in the same range as $K_i$. At this inhibitor concentration the inhibitor-bound enzyme is in equilibrium with the inhibitor-free enzyme. The bulk solution is added to the array of sites, each site having a defined volume (between 10 attoliters and 50 picoliters, more preferably femtoliters). The enzyme concentration is chosen to yield a single enzyme in some sites and no enzyme in other sites. When signal (e.g. fluorescence) is monitored over time, one can see binding, release, and subsequent binding at each site where enzyme is present in the array.

In yet another embodiment, the present invention contempalates a method of detecting a reaction component that affects a reaction, the method comprising: providing a reaction component, a sample and an array, said sample comprising a biomolecule target analyte in a first concentration, said array comprising at least 1000 sites, each site having a defined volume between 10 attoliters and 50 picoliters; diluting said sample to create a diluted sample, said diluted sample comprising a biomolecule target analyte in a second concentration; contacting said array with said diluted sample such that the ratio of biomolecule target analyte to each site of said array is less than 1:5; introducing said reaction component to each site of said array; and determining whether the reaction component affects a reaction.

In still another embodiment, the present invention contemplates a method of detecting enzyme inhibition, the method comprising: providing i) a reaction component, ii) a sample, said sample comprising a enzymatic target analyte in a first concentration, iii) a substrate for said enzymatic target analyte, and iv) an array, said array comprising at least 10,000 sites, each site having a defined femtoliter volume; diluting said sample to create a diluted sample, said diluted sample comprising a enzymatic target analyte in a second concentration; contacting said array, in any order, with said substrate, said reaction component, and said diluted sample such that the ratio of enzymatic target analyte to each site of said array is between 1:5 and 1:500; and determining whether the reaction component inhibits the reaction of said enzyme with said substrate.

In one embodiment, nanoparticles are utilized for the digital readout of target and concentrations. Nanoparticles have several advantages over an enzyme digital readout. With an enzyme approach, as concentrations increase, the linearity of the digital readout, which follows a Poisson distribution, is lost. Using nanoparticles functionalized with fluorescent species, the present invention contemplates in one embodiment to be able to perform a digital readout as well as the traditional bulk intensity readout, which is used extensively in our laboratory. As the linearity of the digital signal is lost, the analysis can be shifted from a digital readout to a traditional intensity readout using the nanoparticles. The signal can then be read as an average intensity of each site in the array (e.g. average well intensity) correlating to concentration, and will significantly increase the range of concentrations that can be read with this system. In some embodiments employing nanoparticles, the array is not sealed.

A variety of embodiments are contemplated, including but not limited to embodiments where the target is attached to the gold particle and introduced into an array, said array comprising a binding partner (e.g. immobilized ligand). In another embodiment, the target analyte may be attached or otherwise immobilized on the array and the gold particle-binding partner conjugate can be introduced to the array to bind the target analyte. In another embodiment, a first binding ligand may immobilize the target, and the gold-particle-second binding ligand may be introduced into the array to bind the target. In one embodiment, the target is nucleic acid and the binding partners are complementary probes. In another embodiment, a two-step sandwich format is contemplated, where the target can be unlabeled and incubated with a first complementary probe; thereafter, the nanoparticle-second probe conjugate is introduced into the site of the array. The target can be viewed to operate as a linker in this embodiment.

**Brief Description of the Drawings**

FIGS. 1a, 1b, and 1c are side view cross-section schematics representing etched bundle modifications, according to one embodiment of the present invention.

FIGS. 2a, 2b, and 2c are side view cross-section schematics representing a sandwich assay, according to one embodiment of the present invention.

FIGS. 3a and 3b are photographs depicting Streptavidin Alexa Fluor 568® binding to (a) an unpolished biotin modified fiber optic array, and (b) a polished biotin modified fiber optic array, according to one embodiment of the present invention.

FIGS. 4a, 4b, 4c, 4d, 4e, and 4f are photographs depicting experiments according to one embodiment of the present invention in which β-galactosidase hydrolyzes RDG to form resorufin. More specifically, each of these figures depicts a different sample having a different concentration of SβG. The concentrations were: (a) 128 amol, (b) 51 amol, (c) 25 amol, (d) 7.5 amol, and (e) 2.6 amol, and (f) was the control.

FIG. 5 is a chart depicting a log-log plt of the moles of target present in a sample with the resulting percentage of active reaction vessels, according to one embodiment of the present invention.

FIG. 6a is a microscopic photograph of an entire fiber array and an inset close-up of the bundle, according to one embodiment of the present invention.

FIG. 6b is an AFM image of a portion of an etched surface, according to one embodiment of the present invention.

FIGS. 7a, 7b, and 7c depict enclosure of the reaction vessels and evaluation of the seal, according to one embodiment. FIG. 7a is a microscopic photograph of a solution of Ru(bpy)$_3$Cl$_2$ enclosed in the array of chambers. FIG. 7b is a microscopic photograph of a small octagonal portion of the bundle photobleached with UV light. FIG. 7c is a microscopic photograph of FIG. 7b taken 60 minutes later.

FIGS. 8a, 8b, and 8c are microscopic photographs depicting detection of the activity of single molecules of β-galactosidase, according to various embodiments of the present invention. FIG. 8a is a microscopic photograph of a background image of a portion of an array. FIG. 8b is a microscopic photograph of an image taken of a portion of a 1:5 enzyme to vessel assay. FIG. 8c is a microscopic photograph of a 1:80 enzyme to vessel assay.

FIG. 9 is a schematic of one embodiment for measuring rate constants of enzymes at the single enzyme level.

FIG. 10 is a representative image of an array wherein signal is detected due to release of inhibitor.

FIGS. 11a, 11b and 11c are time traces showing substrate turnover in the presence of inhibitor or absence (11c) of inhibitor.

FIG. 12 shows the plot of measurements enabling determination of Koff for a particular enzyme/inhibitor pair.

FIGS. 13a and 13b show a plurality of time traces (13a) and a single time trace (13b) plotted to show increased signal upon inhibitor release.

FIG. 14 is a histogram of off-times, permitting a calculation of the half-life values for a particular enzyme/inhibitor pair.

FIG. 15 schematically shows one embodiment wherein nanoparticles are employed to enhance detection.

FIG. 16 schematically shows an alternative embodiment wherein nanoparticles are employed to enhance detection.

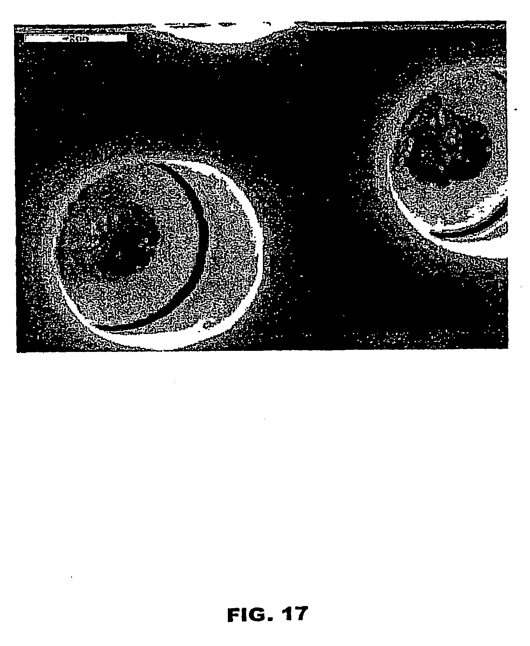

FIG. 17 shows the detection of gold nanoparticles at sites in an array.

**Detailed Description**

The present invention relates to methods for enzymatic detection and quantification of a target analyte or target analytes in a sample. More specifically, the present invention relates to enzymatic detection and quantification of target analytes using arrays of micron- to nanoscale-sized reaction vessels containing capture components. According to one embodiment, an array of reaction vessels containing capture components is contacted with a sample containing at least one target analyte. A chromogenic substrate is then added and the resulting chromogenic product of the enzymatic reaction allows for detection of the analyte. Further, the percentage of reaction vessels with captured target analytes can be used to calculate the amount of target analyte in the sample using a binary readout method.

More specifically, the present invention provides for an array of micron- to nanoscale-sized reaction vessels specifically functionalized and capable of capturing target molecules that are enzymes or enzyme-labelled. The ability to immobilize the target allows the use of washing steps and indirect assays, as outlined below. In use, single enzyme (or enzyme-labelled) molecules are captured in individual reaction vessels and catalyze the production of a sufficient number of chromogenic product molecules to generate a detectable signal. In the present invention, only a portion of the reaction vessels bind a target molecule, thereby enabling a binary readout of target concentration from the array.

Thus, the direct enzymatic amplification in the method and system of the present invention allows for direct amplification of a detectable signal. Further, unlike the prior art methods, the present invention allows for detection of low concentrations of protein.

The quantification method, according to one embodiment, is a novel method for concentration determination based on statistical analysis. The sample enzyme concentration is determined by distributing the enzyme-containing sample and a suitable substrate, into many nanoscale reaction vessels. In this method, the vessels contain either zero or one enzyme molecule. By observing the presence or absence of a fluorescent product resulting from single enzyme molecule catalysis in each reaction vessel, a binary readout method can be used to count enzyme molecules. Finally, the percentage of reaction vessels occupied by enzyme molecules is correlated to the bulk enzyme concentration.

## I. Arrays

The present disclosure provides array compositions comprising at least a first substrate with a surface comprising a plurality of assay locations. By "array" herein is meant a plurality of capture components in an array format. The size of the array will depend on the composition and end use of the array. Arrays containing from about 2 different capture components to many millions can be made, with very large arrays being possible, including very large fiber optic arrays. Generally, the array will comprise from two to as many as a billion or more capture components, depending on the size of the wells and the substrate, as well as the end use of the array, thus very high density, high density, moderate density, low density and very low density arrays may be made. Preferred ranges for very high density arrays are from about 10,000,000 to about 2,000,000,000, with from about 100,000,000 to about 1,000,000,000 being preferred. High density arrays range about 100,000 to about 10,000,000, with from about 1,000,000 to about 5,000,000 being particularly preferred. Moderate density arrays range from about 10,000 to about 50,000 being particularly preferred, and from about 20,000 to about 30,000 being especially preferred. Low density arrays are generally less than 10,000, with from about 1,000 to about 5,000 being preferred. Very low density arrays are less than 1,000, with from about 10 to about 1000 being preferred, and from about 100 to about 500 being particularly preferred. In some embodiments, multiple substrates may be used, either of different or identical compositions. Thus for example, large arrays may comprise a plurality of smaller substrates.

The compositions comprise a substrate. By "substrate", "array substrate" or "solid support" or other grammatical equivalents herein is meant any material that can be modified to contain discrete individual sites appropriate for the attachment or association of target analytes and is amenable to at least one detection method. As will be appreciated by those in the art, the number of possible substrates are very large, and include, but are not limited to, glass and modified or functionalized glass, plastics (including acrylics, polystyrene and copolymers of styrene and other materials, polypropylene, polyethylene, polybutylene, polyurethanes, Teflon™, etc.), polysaccharides, nylon or nitrocellulose, composite materials, ceramics, and plastic resins, silica or silica-based materials including silicon and modified silicon, carbon, metals, inorganic glasses, plastics, optical fiber bundles, and a variety of other polymers. In general, the substrates allow optical detection and do not appreciably fluoresce.

In one embodiment, the substrate comprises the end of an optical fiber bundle. Alternatively, the substrate does not comprise the ends of an optical fiber bundle. For example, the substrate may be a spotted, printed or photolithographic substrate known in the art; see for example WO 95/25116; WO 95/35505; PCT US98/09163; U.S. Patent Nos. 5,700,637; 5,807,522 and 5,445,934; and U.S.S.N.s 08/851,203 and 09/187,289, and references cited within. One advantage of using the distal end of a optical fiber bundle as a substrate in the present invention is that the individual fibers in contact with each well can be used to carry both excitation and emission light to and from the wells, enabling remote interrogation of the well contents. Further, an array of optical fibers provides the capability for simultaneous excitation of molecules in adjacent vessels, without signal "crosstalk" between fibers. That is, excitation light transmitted in one fiber does not escape to a neighboring fiber.

In one embodiment, the substrate is planar, although as will be appreciated by those in the art, other configurations of substrates may be used as well; for example, three dimensional configurations can be used. Preferred substrates include optical fiber bundles as discussed below, and flat planar substrates such as glass, polystyrene and other plastics and acrylics.

In one embodiment, at least one surface of the substrate is modified to contain discrete, individual sites (also referred to herein as "reaction vessels" and "microwells") for later association of target analytes. These sites generally comprise physically altered sites, i.e. physical configurations such as wells or small depressions in the substrate that can retain the beads. The microwells may be formed as is generally known in the art using a variety of techniques, including, but not limited to, photolithography, stamping techniques, molding techniques and microetching techniques. As will be appreciated by those in the art, the technique used will depend on the composition and shape of the substrate.

In one embodiment, physical alterations are made in a surface of the substrate to produce the sites. In a preferred embodiment, the substrate is a fiber optic bundle and the surface of the substrate is a terminal end of the fiber bundle. In this embodiment, wells are made in a terminal or distal end of a fiber optic bundle comprising individual fibers. In this embodiment, the cores of the individual fibers are etched, with respect to the cladding, such that small wells or depressions are formed at one end of the fibers. The required depth of the wells will depend on the size of the beads to be added to the wells. In one aspect of the present invention, the physical alterations can be made as taught in U.S.

Patents 6,023,540, 6,327,410, and 6,858,394.

The sites may be a pattern, i.e. a regular design or configuration, or randomly distributed. A preferred embodiment utilizes a regular pattern of sites such that the sites may be addressed in the X-Y coordinate plane. "Pattern" in this sense includes a repeating unit cell, preferably one that allows a high density of beads on the substrate.

For use in the present invention, the reaction vessels have a volume ranging from about 10 attoliters to about 50 picoliters. Alternatively, the reaction vessels range in size from about 1 femtoliter to about 1 picoliter. In a further alternative, the reaction vessels range from about 30 femtoliters to about 60 femtoliters.

In one aspect of the present invention, the array is a fiber optic array. The array, according to one embodiment, can be made as follows. First, the reaction vessels are formed on the distal end of a fiber optic bundle. According to one embodiment, the vessels are created using an etching process, such as, for example, an acid etching process, resulting in reaction vessels of the desired volume. That is, the etching process creates depressions or holes in the core material at the end of the fiber bundle, while the cladding material is not impacted, thus resulting in reaction vessels. Alternatively, both the core material and cladding material are etched, but the cladding material is etched at a slower rate than the core material, thereby resulting in reaction vessels. One advantage of the fiber optic array format is that it circumvents a complicated microfabrication procedure and provides the ability to observe many reaction vessels simultaneously.

## II. **Capture Components**

The microwells of the present invention comprise at least one capture component. A capture component (also referred to as a "capture binding ligand," "binding ligand," "capture binding species," or "capture probe") is any molecule, compound, or microwell modification that can be used to probe for, attach, bind or otherwise capture a target analyte within a microwell on the substrate, such that the target analyte is immobilized during the assay. Generally, the capture binding ligand or component allows the attachment of a target analyte to the microwell, for the purposes of detection, quantification, or other analysis.

As will be appreciated by those in the art, the composition of the capture component will depend on the composition of the target analyte. Capture components for a wide variety of analytes are known or can be readily found using known techniques. For example, when the analyte is a protein, the capture components or binding ligands include proteins (particularly including antibodies or fragments thereof (FAbs, etc.)) or small molecules. Preferred capture component proteins include peptides. For example, when the analyte is an enzyme, suitable binding ligands include substrates and inhibitors. Antigen-antibody pairs, receptor-ligands, and carbohydrates and their binding partners are also suitable analyte-binding ligand pairs. In addition, when the analyte is a single-stranded nucleic acid, the binding ligand may be a complementary nucleic acid. Similarly, the analyte may be a nucleic acid binding protein and the capture binding ligand is either single-stranded or double stranded nucleic acid; alternatively, the binding ligand may be a nucleic acid-binding protein when the analyte is a single or double-stranded nucleic acid. Alternatively, as is generally described in U.S. Patents 5,270,163, 5,475,096, 5,567,588, 5,595,877, 5,637,459, 5,683,867, 5,705,337, and related patents, nucleic acid "aptomers" can be developed for binding to virtually any target analyte. As will be appreciated by those in the art, any two molecules that will associate may be used, either as an analyte or as the capture component. Similarly, there is a wide body of literature relating to the development of capture components based on combinatorial chemistry methods.

Suitable analyte/capture component pairs include, but are not limited to, antibodies/antigens, receptors/ligands, proteins/nucleic acid, enzymes/substrates and/or inhibitors, carbohydrates (including glycoproteins and glycolipids)/lectins, proteins/proteins, proteins/small molecules; and carbohydrates and their binding partners are also suitable analyte-binding ligand pairs. These may be wild-type or derivative sequences. According to one embodiment, the capture components are portions (particularly the extracellular portions) of cell surface receptors that are known to multimerize, such as the growth hormone receptor, glucose transporters (particularly GLUT 4 receptor), transferring receptor, epidermal growth factor receptor, low density lipoprotein receptor, high density lipoprotein receptor, epidermal growth factor receptor, leptin receptor, interleukin receptors including IL-1, IL-2, IL-3, IL-4, IL-5, IL-6, IL-7, IL-8, IL-9, IL-11, IL-12, IL-13, IL-15, and IL-17 receptors, human growth hormone receptor, VEGF receptor, PDGF receptor, EPO receptor, TPO receptor, ciliary neurotrophic factor receptor, prolactin receptor, and T-cell receptors.

In a preferred embodiment, the capture component is attached to the microwell or reaction vessel as outlined herein, for example via an "attachment component" (also referred to herein as an "attachment linker"). An "attachment component," as used herein, is defined as any component, functionalization, or modification of the microwells that results in the attachment of the capture component, and can include bonds and/or linkers. Alternatively, the capture component may utilize a capture extender component. In this embodiment, the capture component or binding ligand comprises a first portion that will bind the target analyte and a second portion that can be used for attachment to the surface.

The method of attachment of the capture binding ligand to the attachment linker will generally be done as is known in the art, and will depend on the composition of the attachment linker and the capture binding ligand. In general, the capture binding ligands are attached to the attachment linker through the use of functional groups on each that can then be used for attachment. According to one embodiment, the functional group is a chemical functionality. That is, the microwell surface is derivatized such that a chemical functionality is bound to the surface. Preferred functional groups for attachment are amino groups, carboxy groups, oxo groups and thiol groups. These functional groups can then be attached, either directly or through the use of a linker, sometimes referred to herein as a "cross-linker." Linkers are known in the art; for example, homo-or hetero-bifunctional linkers as are well known (see 1994 Pierce Chemical. Company catalog, technical section on cross-linkers, pages 155-200). Preferred linkers include, but are not limited to, alkyl groups (including substituted alkyl groups and alkyl groups containing heteroatom moieties), with short alkyl groups, esters, amide, amine, epoxy groups and ethylene glycol and derivatives being preferred. Linkers may also be a sulfone group, forming sulfonamide.

According to one embodiment, the functional group is a light-activated functional group. That is, the functional group can be activated by light to attach to the target analyte or to the crosslinker. One example is the PhotoLink™ technology available from SurModics, Inc. in Eden Prairie, MN.

In one alternative aspect of the invention, the functional group is added without derivatizing the well surface. That is, the functional groups can be added to the surface by adding a molecule having an attached functional group attached, wherein the molecule has a binding affinity for the well surface. The molecule, according to one embodiment is bovine serum albumin. Alternatively, the molecule is any protein capable of binding or sticking to the vessel surface. In a further alternative, the molecule is any molecule capable of binding or sticking to the vessel surface. In one example, the molecule is bovine serum albumin with free amine groups on its surface. The crosslinker can then be added to attach to the amine groups.

According to one exemplary embodiment in which the capture component is a chemical crosslinker, the target analyte is attached using chemical crosslinking in the following manner. First, the reaction vessel surface is derivatized with a functional group such as $NH_2$. Next, the crosslinker and the target analyte are added to the array such that the crosslinker attaches to the $NH_2$ and the target analyte attaches to the crosslinker. In an alternative embodiment described in further detail below in which the target analyte is not an enzyme, a label having an enzymatic component can also be attached to the target analyte.

In this way, capture binding ligands comprising proteins, lectins, nucleic acids, small organic molecules, carbohydrates, etc. can be added.

One embodiment utilizes proteinaceous capture components or capture binding ligands. As is known in the art, any number of techniques may be used to attach a proteinaceous capture binding ligand. "Protein" in this context includes proteins, polypeptides, peptides, including, for example, enzymes. A wide variety of techniques are known to add moieties to proteins. One preferred method is outlined in U.S. Patent No. 5,620,850. The attachment of proteins to surfaces is known; see also Heller, Acc. Chem. Res. 23:128 (1990), and related work.

An alternative embodiment utilizes nucleic acids as the capture binding ligand, for example for when the target analyte is a nucleic acid or a nucleic acid binding protein, or when the nucleic acid serves as an aptamer for binding a protein, as is well known in the art.

According to one embodiment, each microwell comprises a plurality of capture components. The plurality of capture components, in one aspect of the invention, are distributed on the surface of the well like a "lawn." Alternatively, the capture components are distributed in any known fashion.

The binding between the capture component and the target analyte, in accordance with one embodiment, is specific and the capture component is part of a binding pair. That is, the capture component is a target specific capture component that specifically binds with or has specificity for the target analyte. More specifically, the capture component binds specifically and directly to the target analyte. By "specifically bind" or "binding specificity" herein is meant that the capture component binds the analyte with specificity sufficient to differentiate between the analyte and other components or contaminants of the test sample. For example, the capture component according to one embodiment is an antibody that binds specifically to some portion of the target analyte. The antibody, according to one embodiment, can be any antibody capable of binding specifically to a target analyte. For example, appropriate antibodies include, but are not limited to, monoclonal antibodies, bispecific antibodies, minibodies, domain antibodies, synthetic antibodies (sometimes referred to as "antibody mimetics"), chimeric antibodies, humanized antibodies, antibody fusions (sometimes referred to as "antibody conjugates"), and fragments of each, respectively.

However, as will be appreciated by those in the art, it is possible to detect analytes using binding which is not highly specific; for example, the systems may use different capture components such as, for example, an array of different ligands, and detection of any particular analyte is via its "signature" of binding to a panel of binding ligands, similar to the manner in which "electronic noses" work. This finds particular utility in the detection of chemical analytes. The binding should be sufficient to remain bound under the conditions of the assay, including wash steps to remove non-specific binding. In some embodiments, for example in the detection of certain biomolecules, the binding constants of the analyte to the binding ligand will be at least about $10^4$-$10^6$ M$^{-1}$, with at least about $10^5$ to $10^9$ M$^{-1}$ being preferred and at least about $10^7$-$10^9$ M$^{-1}$ being particularly preferred.

According to one embodiment in which the target analyte is a cell, including, for example, bacterial cells, the capture component is an adhesin receptor molecule. In use, the adhesin receptor molecule binds with a surface protein called an adhesin on the extracellular surface of the target cell, thereby immobilizing or capturing the cell. Alternatively, in embodiments in which the target analyte is another type of cell (a non-bacterial cell), the capture component is an appropriate cell surface receptor that binds the target analyte cell. In a further embodiment in which the target analyte is a cell, the capture component is fibronectin. For example, fibronectin can be used when the target analyte is a nerve cell.

Alternatively, the capture component is a non-specific capture component. That is, the capture component does not bind specifically to a target analyte, but rather binds to a corresponding binding partner associated with or attached to the target analyte. For example, the non-specific capture component according to one embodiment is a chemical cross-linker as described above. According to one embodiment, every peptide molecule in a target sample can attach to the chemical cross-linker. This type of system can be used to identify enzyme target analytes because the analytes are detected by modifying the substrate.

In one example of a non-specific capture component according to one embodiment, the capture component is streptavidin, which binds with high affinity to biotin, and thus binds to any molecule to which biotin has been attached. Alternatively, the capture component is biotin, and streptavidin is attached to or associated with the target analyte such that the target analyte can be captured by the biotin.

According to one embodiment, the capture component is added to the reaction vessels in the following manner. First, the microwells are prepared for attachment of the capture component(s). That is, the microwells are modified or an attachment component is added to the microwells such that the capture component(s) will attach to the microwells. In one embodiment, the microwells are derivatized with a chemical functionality as described above. Next, the capture component is added.

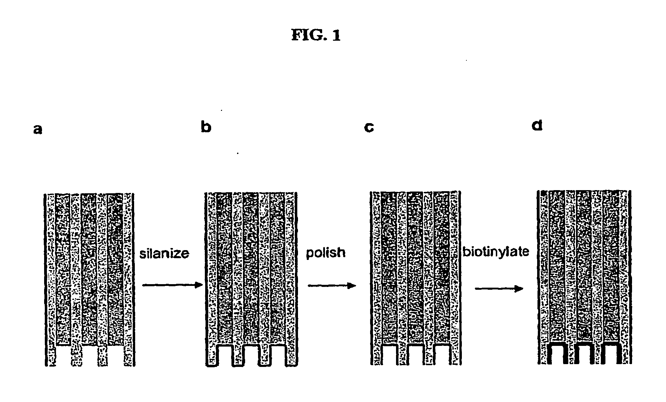

One example of capture component attachment is depicted in FIG. 1, in which reaction vessels of the present invention are functionalized with biotin. As shown in FIG. 1a, the array of the present invention in this example is a fiber optic bundle 10. To attach the capture component 18, the microwells are first modified with an attachment component 16, which in this example is an aminopropyl silane 16 that is bound to both the core 12 and cladding 14 surfaces of the distal end of the fiber bundle 10, as shown in FIG. 1b. The modification with aminopropyl silane is effective in this example because NHS-biotin attaches to an amino-silanized surface 16. However, since the capture component 18 should be present only within the reaction vessels, the external surfaces of the substrate, such as the external surfaces of the cladding 14, should not be silanized. That is, the silanization must be removed from the external cladding surface 14 to avoid biotin attachment. In this example as shown in FIG. 1c, the silanization 16 was removed from the external cladding layer 14 by polishing the amino-silanized fibers for 10 seconds with 0.3 $\mu$m lapping film, thereby removing the top amino-silanized cladding layer.

After the attachment component 16 has been added to the microwells, the capture component 18 can be attached. In the example in FIG. 1, the capture component 18 is biotin 18. As shown in FIG. 1d, biotin succinimidyl ester 18 is attached to the amino groups 16 on the well surfaces 12.

### III. Target Analytes

As discussed herein, the array of the present disclosure provides for detection, quantification, and further analysis of target analytes. By "target analyte" or "analyte" or grammatical equivalents herein is meant any atom, molecule, ion, molecular ion, compound or particle to be either detected or evaluated for binding partners.

According to one embodiment, the target analyte is an enzyme. For example, the enzyme can be an enzyme from any of the six enzyme classifications: oxidoreductases, transferases, hydrolases, lyases, isomerases, and ligases. Thus, appropriate enzymes include, but are not limited to, polymerases, cathepsins, calpains, amino-transferases such as, for example, AST and ALT, proteases such as, for example, caspases, nucleotide cyclases, transferases, lipases, enzymes associated with heart attacks, and the like. When the system of the present disclosure is used to detect viral or bacterial targets, appropriate enzymes include viral or bacterial polymerases and other such enzymes, including viral or bacterial proteases.

Alternatively, the target analyte has an enzymatic component. For example, the target analyte can be a cell having an enzyme or enzymatic component present on its extracellular surface. Alternatively, the target analyte is a cell having no enzymatic component. Such a cell is typically identified using an indirect assaying method described below such as a "sandwich" assay.

In accordance with another embodiment, the target analyte is not an enzyme. As will be appreciated by those in the art, a large number of analytes may be used in the present invention; basically, any target analyte can be used which binds a capture component and/or a secondary binding ligand. As will be explained in further detail below, these target analytes are typically identified using an indirect assay such as a "sandwich" assay. As mentioned above, one suitable target analyte is a cell. In addition, suitable analytes include organic and inorganic molecules, including biomolecules. In a preferred embodiment, the target analyte is a protein. As will be appreciated by those in the art, there are a large number of possible proteinaceous target analytes that may be detected or evaluated for binding partners using the present invention. In addition to enzymes as discussed above, suitable protein target analytes include, but are not limited to, (1) immunoglobulins; (2) hormones and cytokines (many of which serve as ligands for cellular receptors); and (3) other proteins.

According to one embodiment in which the target analyte is not an enzyme and a sandwich assay is performed as described in further detail below, the enzymatic label as described in further detail below can be beta galactosidase. Alternatively, the enzyme label can be, but is not limited to, alkaline phosphatase or horseradish peroxidase.

Further suitable target analytes include, but are not limited to, an environmental pollutant (including pesticides, insecticides, toxins, etc.); a chemical (including solvents, polymers, organic materials, etc.); therapeutic molecules (including therapeutic and abused drugs, antibiotics, etc.); biomolecules (including hormones, cytokines, proteins, nucleic acids, lipids, carbohydrates, cellular membrane antigens and receptors (neural, hormonal, nutrient, and cell surface receptors) or their ligands, etc); whole cells (including procaryotic (such as pathogenic bacteria) and eukaryotic cells, including mammalian tumor cells); viruses (including retroviruses, herpesviruses, adenoviruses, lentiviruses, etc.); and spores; etc.

### IV. Enzymatic Substrate

After the target analyte(s) are captured within the microwell(s) (and after a washing step, according to certain embodiments), a reaction component is added to the array. By "reaction component," as used herein, is meant a molecule that affects an enzymatic reaction when contacted with an enzyme or enzymatic molecule. By "affects" a reaction is meant to include, but is not limited to, inducing, activating, or altering (for example, slowing down or speeding up) a reaction, or inhibiting a reaction. According to one embodiment, the reaction component is a chromogenic enzymatic substrate. A "chromogenic enzymatic substrate" as used herein is any molecule that is converted by an enzyme into a chromogenic product as a result of an enzymatic reaction. "Chromogenic" means relating to color or pigment in the optical (visible light) spectrum and includes fluorogenic.

It is understood in the art that chromogenic substrates are known or can be made for enzymes in any of the six enzyme classifications. Thus, any known chromogenic substrate capable of producing a chromogenic product in a reaction with a particular enzyme can be used in the present invention, including any of the chromogenic enzyme substrates disclosed in The Handbook - A Guide to Fluorescent Probes and Labeling Technologies, Tenth Ed., Chapter 10, (http://probes.invitrogen.com/handbook/sections/1000.html).

According to one embodiment in which the assay of the present invention is a sandwich assay as described further herein in which the enzyme label is beta galactosidase, the substrate added to the array is a beta galactosidase substrate such as resorufin-$\beta$-D-galactopyranoside.

### V. Assay Methods

The array of the present disclosure can be used for several different assay methods. More specifically, the present invention provides for both (a) target analyte detection and (b) quantification of target analyte concentration in a sample.

Generally, the system or array of the present disclosure is exposed to an analyte of interest (or contacted with a sample containing an analyte of interest) and the analyte is immobilized by a capture component in a microwell, under conditions suitable for immobilization of the target analyte to at least one of the capture components, i.e. generally physiological conditions. For purposes of the present application, the term "immobilized" means attached, bound, or affixed to a capture component in a microwell. Thus, the interaction between any analyte molecule and the capture component in a microwell results in immobilization of the analyte molecule within that microwell.

According to one aspect of the invention, the sample of interest is placed in contact with the array of the present disclosure (or the array is incubated in the sample) for a period of from about 45 minutes to about 75 minutes. Alternatively, the array and sample are contacted for a period of from about 50 minutes to about 70 minutes. In a further alternative, the incubation period is about 1 hour.

According to one embodiment, a wash step is performed after contacting the array with the sample. The wash step is intended to wash away any target analytes or non-target molecules that are not bound to a capture component. Alternatively, no wash step is needed.

In one aspect of the invention, a secondary binding ligand is then added to the array. Generally, the secondary binding ligand is added if the assay is an indirect assay such as a "sandwich assay" (when the target analyte is not an enzyme), as described in further detail herein. The secondary binding ligand, as discussed above, will associate with or bind to the bound target analyte and comprises an enzymatic component. The secondary binding ligand is added in an amount sufficient to ensure that a ligand comes into contact with every bound target analyte in the array. Alternatively, no secondary binding ligand is added, such as, for example, when the target analyte is going to be detected directly.

A chromogenic enzymatic substrate as described above is then introduced or added to the array. The chromogenic enzymatic substrate is provided in an amount sufficient to contact any captured target analyte. The chosen substrate reacts with or is modified by the enzymatic component such that the reaction produces a chromogenic product and thus an optical signal. The presence of the chromogenic product in the array can provide information about the identity and/or concentration of an analyte based on the interaction of the analyte with the capture component and the enzymatic substrate (and the secondary binding ligand, in some cases).

In one embodiment of the present invention, the microwells are sealed after the enzymatic substrate is added. That is, a sealing component is placed in contact with the face of the substrate, thereby fluidly isolating each microwell and sealing its contents therein. A "sealing component," as used herein, is defined as any material or device large enough to cover the entire surface of the array substrate and capable of contacting the array substrate surface such that each reaction vessel is sealed or isolated such that the contents of each vessel cannot escape the vessel. According to one embodiment, the sealing component is a silicone elastomer gasket that is placed against the substrate surface with a uniform pressure across the entire substrate. By sealing the contents in each microwell, the enzymatic reaction can proceed within the microwell, thereby producing a detectable amount of the chromogenic product that is retained in the microwell for detection purposes. That is, the enzyme converts the substrate into a chromogenic product that builds up to a locally high concentration in each sealed vessel, generating a detectable chromogenic signal.

According to one embodiment, the present invention provides for a microscope system equipped with a mechanical platform that applies the sealing component. The platform is positioned beneath the microscope stage on the microscopy system. After the assay contents have been added to each well, the sealing component is sandwiched between a flat surface (such as, for example, a microscope slide) and the array substrate using uniform pressure applied by the mechanical platform.

The assays may be run under a variety of experimental conditions, as will be appreciated by those in the art. A variety of other reagents may be included in the screening assays. These include reagents like salts, neutral proteins, e.g. albumin, detergents, etc which may be used to facilitate optimal protein-protein binding and/or reduce non-specific or background interactions. Also, reagents that otherwise improve the efficiency of the assay, such as protease inhibitors, nuclease inhibitors, anti-microbial agents, etc., may be used. The mixture of components may be added in any order that provides for the requisite binding. Various blocking and washing steps may be utilized as is known in the art.

The microwells exhibiting activity or changes in their optical signature may be identified by a conventional optical train and optical detection system. Depending on the particular chromogenic enzymatic substrates used and the operative wavelengths of their chromogenic products, optical filters designed for a particular wavelengths may be employed for optical interrogation of the microwells. In a preferred embodiment, the system or array of the present invention is used in conjunction with an optical fiber bundle or fiber optic array as a substrate.

According to one embodiment, the array of the present disclosure can be used in conjunction with an optical detection system such as the system described in U.S. Application No. 09/816,651. For example, according to one embodiment, the array of the present disclosure is the distal end of a fiber optic assembly comprising a fiber optic bundle constructed of clad fibers so that light does not mix between fibers. As depicted in the 09/816,651 Application, the proximal end of the bundle is received by a z-translation stage and x-y micropositioner.

The optical detection system of U.S. Application No. 09/816,651 operates as follows. Light returning from the distal end of the bundle is passed by the attachment to a magnification changer which enables adjustment of the image size of the fiber's proximal or distal end. Light passing through the magnification changer is then shuttered and filtered by a second wheel. The light is then imaged on a charge coupled device (CCD) camera. A computer executes imaging processing software to process the information from the CCD camera and also possibly control the first and second shutter and filter wheels.

The array or system of the present disclosure may be attached to the distal end of the optical fiber bundle using a variety of compatible processes. Wells are formed at the center of each optical fiber of the bundle. Thus, each optical fiber of the bundle conveys light from the single microwell formed at the center of the fiber's distal end. This feature is necessary to enable the interrogation of the optical signature of individual microwells to identify reactions in each microwell. Consequently, by imaging the end of the bundle onto the CCD array, the optical signatures of the microwells are individually interrogatable.

### A. *Detection*

In one aspect of the present disclosure, the present array can be used to detect the presence of a target analyte in a sample. More specifically, the invention provides a method for detecting the product of the enzymatic reaction as an indication of the presence of the target analyte.

The method of detection can proceed either directly or indirectly. If the target analyte is an enzyme, the analyte can be identified by a direct method of detection. Alternatively, if the target analyte is not an enzyme and thus cannot produce a chromogenic product in the presence of a chromogenic enzymatic substrate, the analyte is identified by an indirect method of detection.

The direct method of detection, which involves a target analyte that is an enzyme, proceeds as follows. First, the sample of interest and the array are placed in contact as described in further detail above under suitable conditions. Subsequently, the chromogenic enzymatic substrate is added.

The presence or absence of the target analyte in any given microwell is then detected by optical interrogation. That is, any change in the optical signal caused by production of a chromogenic product is detected. In any microwell containing the target analyte, the analyte modifies or acts upon the substrate in some way, thereby resulting in the release of a chromogenic product, resulting in a change in the optical signal from the microwell. The chromogenic reaction product is then optically detected.

In one embodiment of the present invention, the microwells are sealed after the enzymatic substrate is added, as described above.

The indirect methods of detection involve a target analyte that does not have enzymatic properties. Two indirect methods that can be used with the present invention are the "sandwich" assay and the "competitive" assay.

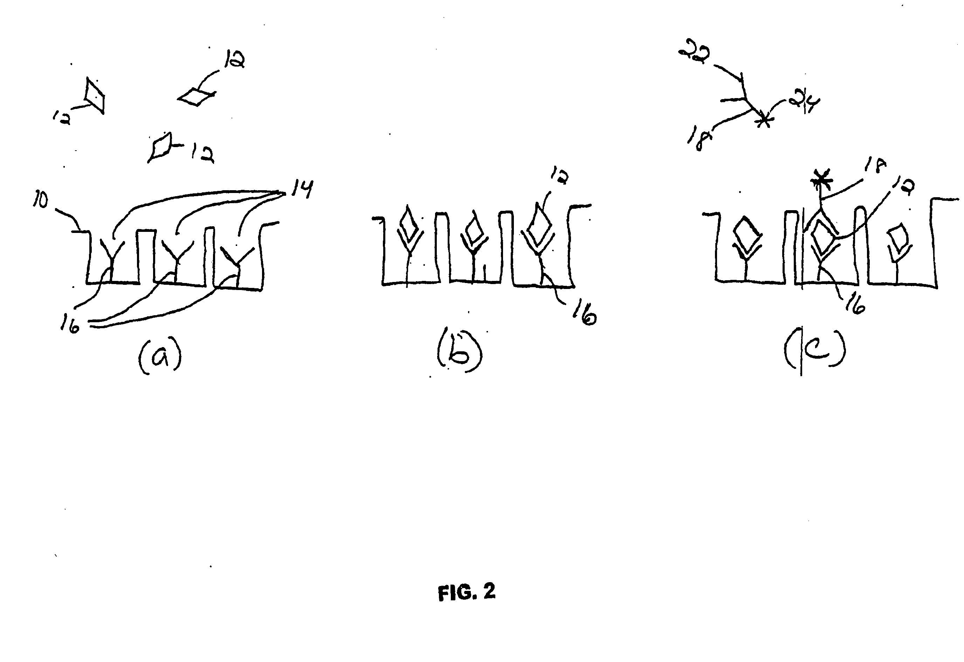

A sandwich assay can be performed as depicted in FIG. 2. First, the sample of interest and the array 10 are placed in contact as shown in FIG. 2a and as described in further detail above. Under suitable conditions, target analyte 12 present in the sample is captured by the capture components 16 in the microwells 14, as shown in FIG. 2b. According to one embodiment, a wash step is then performed.

Next, a solution binding ligand 18 (also referred to herein as a "secondary binding ligand") is added to the array 10, as shown in fig. 2c. Solution binding ligands 18 are similar to capture components 16 in that they bind to target analytes 12. The solution binding ligand 18 may be the same or different from the capture binding ligand 16. The binding of the solution binding ligand 18 to a captured target analyte 12 forms a "sandwich" of sorts. In the absence of the target analyte, the solution binding ligand 18 is washed away.

A solution binding ligand 18 has two components - a binding component 22 and an enzymatic label 24. The binding component 22 is the portion of the solution binding ligand 18 that binds to the target analyte 12. Typically, the solution binding ligand 18 binds to a different portion of the target analyte 12 than the capture component 16, because if both the capture component 16 and solution binding ligand 18 were to bind to the same portion, the solution binding ligand 18 would not be capable of binding to a captured target analyte 12. Thus, the chosen secondary binding ligand 18 can bind to the target analyte 12 while the target analyte 12 is bound to a microwell 14 via a capture component 16.

The enzymatic label 24 is the portion of the solution binding ligand 18 that exhibits enzymatic activity. According to one embodiment, the enzymatic label 24 is an enzyme attached to the solution binding ligand 18.

Subsequently, the chromogenic enzymatic substrate is added.

In one embodiment of the present invention, the microwells are sealed after the enzymatic substrate is added, as described above.

The presence or absence of the target analyte in any given microwell is then detected by optical interrogation. That is, any change in the optical signal caused by production of a chromogenic product is detected. In any microwell containing the target analyte and the secondary binding ligand, the enzyme associated with the secondary binding ligand modifies or acts upon the substrate in some way, thereby producing a chromogenic product, resulting in a change in the optical signal from the microwell. The product is then optically detected.

The competitive assay operates as follows. First, a labelled molecule is added to the array of the present invention, wherein the label is a enzyme or enzymatic component. In this embodiment, the chosen labelled molecule binds with the capture component such that the addition of the labelled molecule to the array results in labelled molecules being bound to capture components in the microwells.

Next, the sample of interest and the array are placed in contact as described in further detail above. The presence of the target analyte in the array causes the displacement of the labelled molecule and binding of the analyte to the capture components. The displacement occurs for the following reason: in this embodiment, the chosen capture component is capable of binding to either of the labelled molecule or the target analyte, thus resulting in a competitive binding situation. As a result, if a labelled molecule is bound to a capture component in a microwell and a target analyte is added, the target analyte will displace the labelled molecule under suitable conditions.

According to one embodiment, a wash step is then performed to remove any non-bound labelled molecules from the array.

Subsequently, the chromogenic enzymatic substrate is added. And as discussed above, according to one aspect of the invention, the microwells are sealed after the enzymatic substrate is added. Alternatively, the microwells are not sealed.

The presence or absence of the target analyte in any given microwell is then detected by optical interrogation. But unlike the optical interrogations that are described above, in this interrogation it is the lack of a chromogenic product that indicates the presence of the target analyte in the microwell. In any microwell containing the target analyte, no enzymatic action occurs and no change occurs in the optical signal from the microwell. In contrast, in any microwell in which the labelled molecule is still present, an optical signal is detected.

In an alternative version of the competitive assay embodiment, both the labelled molecule and sample of interest are added to the array at the same time in fixed volumes. In this version, the target analyte and labelled molecule compete directly for the binding sites on the capture components.

### 1. Subpopulations of identical capture components to same target analyte

In accordance with one detection embodiment, sensor redundancy is used. In this embodiment, a plurality of reaction vessels comprising identical capture components referred to as "subpopulations" are used. That is, each subpopulation comprises a plurality of identical capture components present in microwells of the array. Further, according to one embodiment, each subpopulation comprises a plurality of microwells comprising identical capture components. By using a number of identical capture components for a given array, the optical signal from each microwell can be combined for the subpopulation and any number of statistical analyses run, as outlined below. This can be done for a variety of reasons. For example, in time varying measurements, redundancy can significantly reduce the noise in the system. For non-time based measurements, redundancy can significantly increase the confidence of the data.

The number of subpopulations, according to one embodiment, can range from 2 to any number of subpopulations possible given the limitations of any known array and the number of different capture components. Alternatively, the number can range from about 2 to about 10. In a further alternative, the number can range from about 2 to about 5.

In one embodiment, a plurality of identical capture components are used. As will be appreciated by those in the art, the number of identical capture components in a subpopulation will vary with the application and use of the sensor array. In general, anywhere from 2 to thousands of identical capture components may be used in a given subpopulation, with from 2 to 100 being preferred, 2 to 50 being particularly preferred and from 5 to 20 being especially preferred. In general, preliminary results indicate that roughly 10 identical capture components in a subpopulation gives a sufficient advantage, although for some applications, more identical capture components can be used.

Once obtained, the optical response signals from a plurality of microwells within each subpopulation (that is, having the same capture component) can be manipulated and analyzed in a wide variety of ways, including baseline adjustment, averaging, standard deviation analysis, distribution and cluster analysis, confidence interval analysis, mean testing, etc.

### 2. Multiple different capture components to same target analyte

In addition to the sensor redundancy, the array of the present disclosure according to one embodiment utilizes a plurality of capture components that are directed to a single target analyte but are not identical. This embodiment provides for more than one different capture component in each microwell or different capture components in different microwells. In one example, a single target analyte may be provided to which two or more capture components are capable of binding. This adds a level of confidence as non-specific binding interactions can be statistically minimized. In this embodiment, when proteinaceous target analytes are to be evaluated, preferred embodiments utilize capture components that bind to different parts of the target. For example, when two or more antibodies (or antibody fragments) to different portions of the same target protein are used as capture components, preferred embodiments utilize antibodies to different epitopes. Similarly, when nucleic acid target analytes are to be evaluated, the redundant nucleic acid probes may be overlapping, adjacent, or spatially separated. However, it is preferred that two probes do not compete for a single binding site, so adjacent or separated probes are preferred.

In this embodiment, a plurality of different capture components may be used, with from about 2 to about 20 being preferred, and from about 2 to about 10 being especially preferred, and from 2 to about 5 being particularly preferred, including 2, 3, 4 or 5. However, as above, more may also be used, depending on the application.

### 3. Multiple different capture components to multiple target analytes

According to another embodiment, the array of the present disclosure utilizes a plurality of different capture components that are directed to a plurality of target analytes. This embodiment includes more than one different capture component in each microwell or different capture components in different microwells. In one example, two or more target analytes may be provided to which two or more capture components in the same microwells or different microwells are capable of binding.

In this embodiment, more than one target analyte can be identified. For example, two or more target analytes can be identified so long as each different analyte is a different enzyme or has a different enzymatic component such as a enzymatic surface molecule. In one embodiment, the target analytes are identified using multiple enzymatic substrates wherein each substrate produces a different color upon interaction with the appropriate enzyme. Thus, each target analyte can be distinguished based on the color produced by reaction with the substrate. In an alternative embodiment, the target analytes are identified using multiple substrates that each produce the same color. Thus, each target analyte can be distinguished by added the substrates sequentially.

In this embodiment, a plurality of different capture components may be used, with from about 2 to about 20 being preferred, and from about 2 to about 10 being especially preferred, and from 2 to about 5 being particularly preferred, including 2, 3, 4 or 5. However, as above, more may also be used, depending on the application.

Please note that each of the different assay configurations above, including the capture component subpopulations directed to different target analytes and the plurality of capture components directed to the same analyte, can also be utilized for quantification as described below.

### B. *Quantification*

According to the present invention, the present array is used for quantification of the analyte in the sample. That is, there is a correlation between the percentage of reaction vessels containing target analytes and the concentration of the analyte in the sample. Thus, the quantification method of the present invention allows for calculation of the amount of a target analyte in a sample based on the percentage of microwells that captured a target analyte.

Without being limited by theory, the quantification method is driven in part by the fact that the number and volume of reaction vessels employed govern the dynamic range of concentrations that can be determined by this technique. That is, based on the number and volume of the reaction vessels in an array of the present invention, an estimate can be made of the range of concentrations of target analyte in solution that allow for the concentration to be determined using the method of the present invention.

For example, for an array as disclosed in Example 2 with reaction vessels each having a volume of 46 fL, a solution having a concentration of $3.6 \times 10^{-11}$ M $\beta$-galactosidase will yield, on average, one enzyme molecule per vessel. However, it is important to note that distributing a solution having a target analyte concentration within the appropriate range into an array of reaction vessels will not result in the distribution of exactly one enzyme molecule per vessel; statistically, some vessels will have multiple molecules while others will have zero. In the case where the number of enzyme molecules per vessel is high, the data can be fit to a Gaussian distribution. As the ratio of enzyme molecules to reaction vessels approaches zero, the Poisson distribution applies. This limiting distribution is used to calculate the probability of rare events occurring in a large number of trials. For example, based on Poisson statistics, for a concentration of $3.6 \times 10^{-11}$ M, a distribution between zero and five enzyme molecules per container is observed, with the most probable values being zero and one.

Equation 1 can be used to determine the probability of observing $v$ events based on the expected average number of events per trial, $\mu$

$$\text{Equation 1:} \quad P_\mu(v) = e^{-\mu}\,(\mu^v/v!)$$

If the concentrations used are much less than $3.6 \times 10^{-11}$ M, the expected average becomes exceptionally low, the distribution is narrowed, and the probability of observing anything other than 0 or 1 events per trial is improbable in all experimental cases. At these low concentrations, the relationship between the percentage of active reaction vessels and the bulk enzyme concentration is approximately linear. Thus, based on this knowledge, the array of the present invention can be used to determine the concentration of a target analyte in a sample by a simple digital readout system as described herein.

According to one embodiment, the quantification method of the present invention can be performed as follows. The method is a digital readout system (also referred to as a "binary readout system") that includes first detecting the target analytes in the array of microwells by any detection method described above. The number of reaction vessels is then counted and a percentage of the total number of reaction vessels is calculated. That is, utilization of a yes or no response, in conjunction with the high-density array of reaction vessels, permits the digital readout of bulk concentrations of $\beta$-galactosidase. This readout is accomplished by counting the vessels containing an active enzyme molecule across the array, with the resulting "active well" percentage correlating to the enzyme concentration. Given the large number of vessels simultaneously interrogated in the array of the present invention, the ratio of enzyme molecules to reaction vessels could be as low as 1:500, as the large number of wells provides a statistically significant signal even at this low ratio.

Without being limited by theory, it is believed that the quantification method of the present invention is only limited by the number of individual reaction vessels that can be viewed with an acceptable resolution. Thus, expanding the number of vessels that are interrogated by using higher density CCD chips will decrease the limit of detection as the lower limit is defined by the statistics of the small number of active wells that light up at the lower target concentrations. On the other hand, the upper limit of the dynamic range is controlled by the well-to-well deviation from a binary readout. As target concentrations are increased, the binary readout is lost, as a Gaussian distribution becomes a better approximation of target molecule binding. Higher concentrations of target lead to a broad distribution in the number of enzyme molecules that can occupy each well, and consequently, the transition to a non-linear increase in the percentage of active wells.

The limitations of this technique are realized above and below the thresholds of the dynamic range. As the concentration goes below the lower limit of the dynamic range, the number of enzyme molecules is too low to observe sufficient occupied wells and, therefore, the number of wells must be increased in order to make sure that a statistically significant number of them are occupied by enzyme molecules. Results for extremely dilute concentrations have large relative errors associated with them, due to the very small number of reaction vessels that are expected to show activity. Slight deviation from the expected Poisson value, in this case, will result in a large error. The ultimate upper limit to this technique occurs when 100% of the reaction vessels contain at least one enzyme molecule. At this limit, discrimination between two solutions of high enzyme concentrations is not feasible. As the percentage of active vessels approaches 100%, the linearity between concentration and active vessel percentage is lost. This situation results in a broadening distribution, as a normal distribution becomes an increasingly better approximation of the results.

In one aspect of the present invention, the array can also be used to analyze enzyme kinetics. "Enzyme kinetics" as used herein refers to the study of the rates of enzyme-controlled reactions. It is understood in the art of enzyme kinetics that the rate of an enzymatic reaction at low substrate concentrations is proportional to the substrate concentration (is "substrate dependent"). This is referred to as first order. It is further understood that the rate of the reaction at high substrate concentrations reaches a maximum rate and is independent of substrate concentration because the reaction becomes saturated. Thus, if reaction velocity is plotted as a function of substrate concentration, the line initially increases linearly with an increase in substrate and then begins to level off as substrate concentration approaches saturation.

Thus, according to one embodiment, the kinetics of any particular enzyme can be studied using the present system and array. Reaction velocity varies across enzymes for various reasons, including, for example, reaction inhibition caused by allosteric inhibition. The array of the present disclosure allows for study of these varied kinetic characteristics.

According to one embodiment, kinetics are examined in the following fashion. The target analyte.is allowed to bind to the capture component, the substrate is added, and the reaction vessel is sealed. Given that a finite amount of substrate is present in the reaction vessel and that no further substrate can be added due to the sealing of the vessel, the reaction velocity can be determined based on the amount of chromogenic product detected over time.

## C. *Enhancing Signal And Sensitivity*

The one embodment, the present invention contemplates a metallic or semiconductor nanoparticle attached to a biomolecule, and preferably a plurality of biomolecules (e.g. oligonucleotides) in order to enhance signal and sensitivity for the various embodiments of single molecule detection and quantification describe above. In one embodiment, the biomolecules (e.g. oligonucleotides) are labeled with fluorescent molecules at the ends not attached to the nanoparticle. It is not intended that the present invention be limited to the type of nanoparticle or to the use of merely one type of nanoparticle in an assay. In some embodiments, two, or more different types or sizes of nanoparticle are employed. For example, because different sized (e.g., 50 nm and 100 nm diameter) gold nanoparticles scatter light of different colors (due to their different surface plasmon resonances), they can be used to label different DNA targets hybridized to the same array.

Nanoparticles useful in the practice of the invention include metal (e.g., gold, silver, copper and platinum), semiconductor (e.g., CdSe, CdS, and CdS or CdSe coated with ZnS) and magnetic (e.g., ferromagnetite) colloidal materials. Methods of making metal, semiconductor and magnetic nanoparticles are well-known in the art. See, e.g., Schmid, G. (ed.) Clusters and Colloids (VCH, Weinheim, 1994); Hayat, M. A. (ed.) Colloidal Gold: Principles, Methods, and Applications (Academic Press, San Diego, 1991); Massart, R., IEEE Taransactions On Magnetics, 17, 1247 (1981); Ahmadi, T. S. et al., Science, 272, 1924 (1996); Henglein, A. et al., J. Phys. Chem., 99, 14129 (1995); Curtis, A. C., et al., Angew. Chem. Int. Ed. Engl., 27, 1530 (1988). Other nanoparticles useful in the practice of the invention include ZnS, ZnO, $TiO_2$, AgI, AgBr, $HgI_2$, PbS, PbSe, ZnTe, CdTe, $In_2S_3$, $In_2Se_3$, $Cd_3P_2$, $Cd_3As_2$, InAs, and GaAs. See, e.g., Weller, Angew. Chem. Int. Ed. Engl., 32,41 (1993); Henglein, Top. Curr. Chem., 143, 113 (1988); Henglein, Chem. Rev., 89, 1861 (1989); Brus, Appl. Phys. A., 53, 465 (1991); Bahncmann, in Photochemical Conversion and Storage of Solar Energy (eds. Pelizetti and Schiavello 1991), page 251; Wang and Herron, J. Phys. Chem., 95, 525 (1991); Olshavsky et al., J. Am. Chem. Soc., 112, 9438 (1990); Ushida et al., J. Phys. Chem., 95, 5382 (1992). Suitable gold nanoparticles are also commercially available from, e.g., Ted Pella, Inc., Amersham Corporation and Nanoprobes, Inc.

There are methods for controlling nanoparticle growth. U.S. Patent 7,033,415 to Mirkin et al. describes new types of plasmon-driven growth mechanisms for silver nanostructures involving the fusion of triangular nanoprisms. This mechanism, which is plasmon excitation-driven and highly cooperative, produces bimodal particle size distributions. In these methods, the growth process can be selectively switched between bimodal and unimodal distributions using dual beam illumination of the nanoparticles. This type of cooperative photo-control over nanostructure growth enables synthesis of monodisperse nanoprisms with a preselected edge length in the 30-120 nm range simply by using one beam to turn off bimodal growth and the other (varied over the 450-700 nm range) for controlling particle size. While a variety of sizes are contemplated, the size of the nanoparticles is preferably from about 5 nm to about 150 nm (mean diameter), more preferably from about 5 to about 50 nm, most preferably from about 10 to about 30 nm. The nanoparticles may also be rods.

Presently preferred for use in detecting nucleic acids are gold nanoparticles. See U.S. Patent No. 7,169,556. Gold colloidal particles have high extinction coefficients for the bands that give rise to their beautiful colors. These intense colors change with particle size, concentration, interparticle distance, and extent of aggregation and shape (geometry) of the aggregates, making these materials particularly attractive for colorimetric assays. For instance, hybridization of oligonucleotides attached to nanoparticles with oligonucleotides and nucleic acids results in an immediate color change visible to the naked eye.

Gold nanoparticles are also presently preferred for use in nanofabrication for the same reasons given above and because of their stability, ease of imaging by electron microscopy, and well-characterized modification with thiol functionalities. Also preferred for use in nanofabrication are semiconductor nanoparticles because of their unique electronic and luminescent properties.

Alternatively, in one embodiment, the present invention contemplates the use of nanocrystals as imaging agents, and/or coding agents (i.e. agents which permit the detection and/or identification of compounds, including biomolecules, introduced into in the arrays) as well as substances to be transferred themselves in the sites of the arrays. In some embodiments, detection of single molecules within an array is enhanced by the use of nanocrystals. In one embodiment, the nanocrystals are attached or bind to the target analyte. In another embodiment, nanocrystals are attached to a binding partner, the binding partner in turn binding the target analyte. In a further embodiment, nanocrystals are attached to an inhibitor, a substrate or other ligand. In other embodiments, the nanocrystals are used with fiber-optic sensing techniques. In some embodiments of the present invention, imaging modules comprise surface modifications of quantum dots (See e.g., Chan and Nie, Science 281:2016 (1998)) such as zinc sulfide-capped cadmium selenide coupled to biomolecules (Sooklal, Adv. Mater., 10:1083 (1998)).

The terms "semiconductor nanocrystal," "quantum dot" and "Qdot.TM. nanocrystal" are used interchangeably herein and refer to an inorganic crystallite between about 1nm and about 1000 nm in diameter or any integer or fraction of an integer therebetween, preferably between about 2 nm and about 50 nm or any integer or fraction of an integer therebetween, more preferably about 2 nm to about 20 nm (such as about 2, 3, 4, 5, 6, 7, 8, 9, 10, 11, 12, 13, 14, 15, 16, 17, 18, 19, or 20 nm). A semiconductornanocrystal is capable of emitting electromagnetic radiation upon excitation (i.e., the semiconductor nanocrystal is luminescent) and includes a "core" of one or more first semiconductor materials, and may be surrounded by a "shell" of a second semiconductor material. A semiconductor nanocrystal core surrounded by a semiconductor shell is referred to as a "core/shell" semiconductor nanocrystal. The surrounding "shell" material will preferably have a bandgap energy that is larger than the bandgap energy of the core material and may be chosen to have an atomic spacing close to that of the "core" substrate. The core and/or the shell can be a semiconductor material including, but not limited to, those of the group II-VI (ZnS, ZnSe, ZnTe, CdS, CdSe, CdTe, HgS, HgSe, HgTe, MgS, MgSe, MgTe, CaS, CaSe, CaTe, SrS, SrSe, SrTe, BaS, BaSe, BaTe, and the like) and III-V (GaN, GaP, GaAs, GaSb, InN, InP, InAs, InSb, and the like) and IV (Ge, Si, and the like) materials, and an alloy or a mixture thereof.

A semiconductor nanocrystal is, optionally, surrounded by a "coat" of an organic capping agent. The organic capping agent may be any number of materials, but has an affinity for the semiconductor nanocrystal surface. In general, the capping agent can be an isolated organic molecule, a polymer (or a monomer for a polymerization reaction), an inorganic complex, and an extended crystalline structure. The coat is used to convey solubility, e.g., the ability to disperse a coated semiconductor nanocrystal homogeneously into a chosen solvent, functionality, binding properties, or the like. For example, binding properties can be introduced such that the nanocrystal(s) bind to the target analyte of choice. In addition, the coat can be used to tailor the optical properties of the semiconductor nanocrystal. Methods for producing capped semiconductor nanocrystals are discussed in U.S. Patent No. 6,274,323.

In yet other embodiments, detection of single molecules within an array is enhanced by the use of dendrimers. In one embodiment, the dendrimers are attached or bind to the target analyte. In another embodiment, dendrimers are attached to a binding partner, the binding partner in turn binding the target analyte. In a further embodiment, dendrimers are attached to an inhibitor, a substrate or other ligand. In other embodiments, dendrimers are used with fiber-optic sensing techniques (See, e.g., Ye et al., U.S. Pat. App. No. 20040131322, PCT App. No. WO/2004057386, and Thomas et al.,, Biophysical Journal, 86(6), 3959 (2004)).

Dendrimeric polymers have been described extensively (See, Tomalia, Advanced Materials 6:529 (1994); Angew, Chem. Int. Ed. Engl., 29:138 (1990)).

Dendrimer polymers are synthesized as defined spherical structures typically ranging from 1 to 20 nanometers in diameter. Molecular weight and the number of terminal groups increase exponentially as a function of generation (the number of layers) of the polymer Different types of dendrimers can be synthesized based on the core structure that initiates the polymerization process.

The dendrimer core structures dictate several characteristics of the molecule such as the overall shape, density and surface functionality (Tomalia et al., Chem. Int. Ed. Engl., 29:5305 (1990)). Spherical dendrimers can have ammonia as a trivalent initiator core or ethylenediamine (EDA) as a tetravalent initiator core. Recently described rod-shaped dendrimers (Yin et al., J. Am. Chem. Soc., 120:2678 (1998)) use polyethylene-imine linear cores of varying lengths; the longer the core, the longer the rod. Dendritic macromolecules are available commercially in kilogram quantities and are produced under current good manufacturing processes (GMP) for biotechnology applications.

Dendrimers may be characterized by a number of techniques including, but not limited to, electrospray-ionization mass spectroscopy, $^{13}$C nuclear magnetic resonance spectroscopy, $^{1}$H nuclear magnetic resonance spectroscopy, high performance liquid chromatography, size exclusion chromatography with multi-angle laser light scattering, ultraviolet spectrophotometry, capillary electrophoresis and gel electrophoresis. These tests assure the uniformity of the polymer population and are important for monitoring quality control of dendrimer manufacture for GMP applications.

Numerous U.S. Patents describe methods and compositions for producing dendrimers. Examples of some of these patents are given below in order to provide a description of some dendrimer compositions that may be useful in the present invention, however it should be understood that these are merely illustrative examples and numerous other similar dendrimer compositions could be used in the present invention.

U.S. Pat. No. 4,507,466, U.S. Pat. No. 4,558,120, U.S. Pat. No. 4,568,737, and U.S. Pat. No. 4,587,329 each describe methods of making dense star polymers with terminal densities greater than conventional star polymers. These polymers have greater/more uniform reactivity than conventional star polymers, i.e. 3rd generation dense star polymers. These patents further describe the nature of the amidoamine dendrimers and the 3-dimensional molecular diameter of the dendrimers.

U.S. PaL No. 4,631,337 describes hydrolytically stable polymers. U.S. Pat. No. 4,694,064 describes rod-shaped dendrimers. U.S. Pat. No. 4,713,975 describes dense star polymers and their use to characterize surfaces of viruses, bacteria and proteins including enzymes. Bridged dense star polymers are described in U.S. Pat. No. 4,737,550. U.S. Pat. No. 4,857,599 and U.S. Pat. No. 4,871,779 describes dense star polymers on immobilized cores useful as ion-exchange resins, chelation resins and methods of making such polymers.

U.S. Pat. No. 5,338,532 is directed to starburst conjugates of dendrimer(s) in association with a pharmaceutical or other material. This patent describes the use of dendrimers to provide means of delivery of high concentrations of carried materials per unit polymer, controlled delivery, targeted delivery and/or multiple species such as e.g., drugs antibiotics, general and specific toxins, metal ions, radionuclides, signal generators, antibodies, interleukins, hormones, interferons, viruses, viral fragments, pesticides, and antimicrobials.

U.S. Pat. No. 6,471,968 describes a dendrimer complex comprising covalently linked first and second dendrimers, with the first dendrimer comprising a first agent and the second dendrimer comprising a second agent, wherein the first dendrimer is different from the second dendrimer, and where the first agent is different than the second agent.

Other useful dendrimer type compositions are described in U.S. Pat. No. 5,387,617, U.S. Pat. No. 5,393,797, and U.S. Pat. No. 5,393,795 in which dense star polymers are modified by capping with a hydrophobic group capable of providing a hydrophobic outer shell. U.S. Pat. No. 5,527,524 discloses the use of amino terminated dendrimers in antibody conjugates.

The use of dendrimers as metal ion carriers is described in U.S. Pat. No. 5,560,929. U.S. Pat. No. 5,773,527 discloses non-crosslinked polybranched polymers having a comb-burst configuration and methods of making the same. U.S. Pat. No. 5,631,329 describes a process to produce polybranched polymer of high molecular weight by forming a first set of branched polymers protected from branching; grafting to a core; deprotecting first set branched polymer, then forming a second set of branched polymers protected from branching and grafting to the core having the first set of branched polymers, etc.

U.S. Pat. No. 5,898,005 and U.S. Pat No. 5,861,319 describe specific immunobinding assays for determining concentration of an analyte. U.S. Pat. No. 5,661,025 provides details of a self-assembling polynucleotide delivery system comprising dendrimer polycation to aid in delivery of nucleotides to target site. This patent provides methods of introducing a polynucleotide into a eukaryotic cell in vitro comprising contacting the cell with a composition comprising a polynucleotide and a dendrimer polyeation non-covalently coupled to the polynucleotide. Each of these patents is hereby incorporated by reference.

Dendrimer-antibody conjugates for use in in vitro diagnostic applications has previously been demonstrated (Singh et al., Clin. Chem., 40:1845 (1994)), for the production of dendrimer-antibody constructs. Dendrimers have also been conjugated to fluorochromes or molecular beacons and shown to enter cells. They can then be detected within the cell in a manner compatible with sensing apparatus for evaluation of physiologic changes within cells (Baker et al., Anal. Chem. 69:990 (1997)). Finally, dendrimers have been constructed as differentiated block copolymers where the outer portions of the molecule may be digested with either enzyme or light-induced catalysis (Urdea and Horn, Science 261:534 (1993)).

In one embodiment, the present invention contemplates the use of dendrimers with attached imaging agents, e.g. imaging agents such as fluorophores (e.g. fluorescein isothiocyanate) or quantum dots. Fluorescein is easily attached to the dendrimer surface via the isothiocyanate derivatives, available from Molecular Probes, Inc. Hence, the present invention provides a multifunctional dendrimers to enhance single molecule detection.

## VI. Exemplary Uses of the Present Invention

The system and array of the present disclosure has many uses. For example, the array has application to fundamental enzymology studies, as well as digital concentration measurements. Further, the array permits studies with multiple different enzymes and extends the limits of ultra-low detection for protein and DNA targets. With the ability to simultaneously monitor a large array of reaction vessels, single molecule enzymology can be used to resolve individual enzyme molecule behavior (e.g. Koff and Kon) from bulk kinetic signal.

Another use, for example, is environmental monitoring of bacteria or viruses or both. An environmental sample potentially containing certain bacteria can be placed in contact with an array of the present invention. To detect the bacteria, the bacteria cells are lysed and a bacterial enzyme (or more than one enzyme) is targeted for detection. According to one embodiment, the cells are lysed prior to being added to the array. Alternatively, the cells are captured and a lysing step occurs on the array prior to detection. In a further alternative, no lysis may be necessary if a cell surface marker is targeted. For example, the bacteria or virus of interest can be captured with an antibody that is specific to a surface marker on the target, and then the capture can be detected with a sandwich-type assay by adding an enzyme-labelled antibody that binds to the target in another location.

Another use, for example, involves measuring gene expression. As noted above, in one embodiment, the target analytes are cells and these are introduced into the sites of the array under conditions such that some sites contain single cells. This is the first step in measuring gene expression, which can be done if desired at the whole cell level by detecting surface proteins and secreted proteins. Alternatively, the cells can be lysed and transcripts (or other components) from the cells addressed. In one embodiment, each site contains one or no transcripts. In one embodiment, normal and cancer cells are compared. In one embodiment, the cell to cell variation in gene expression can be addressed by examining gene expression at the single cell level.

### EXAMPLES

### Example 1

In this example, a proof-of-concept binding assay is performed using enzymatic signal amplification in an array of femtoliter sized reaction vessels. More specifically, various assays are performed to detect varying amounts of streptavidin-β-galactosidase (SβG) in solution using a biotinylated array of the present disclosure and then the correlation between the number of wells with captured SβG molecules and the concentration of the SβG in the sample is examined.

In this example, an etched fiber optic array is used to create a collection of ferntoliter sized reaction vessels, each specifically functionalized and capable of capturing enzyme-labeled target molecules. Single enzyme molecules are confined to individual reaction vessels and catalyze the production of a sufficient number of fluorescent product molecules to generate a positive signal. At low target molecule concentrations, only a percentage of the capture sites bind a target molecule, enabling a binary readout of target concentration from the high-density array.

### Materials

The reactor vessel arrays in this example are generated using an acid etch of the distal face of a polished 1mm fiber optic array, consisting of 24,000 individual 4.5 $\mu$m optical fibers. The core fiber material is silica, and the cladding around each fiber is germania-doped silica, which etches at a slower rate. The 4.5 $\mu$m fibers are etched to a depth of 2.9 $\mu$m, creating an array of reactor vessels, each with a 46 fL volume *(see* Figure 1a).

The fibers were first modified with an aminopropyl silane bound to both the core and cladding surfaces *(see* Figure 1b). To avoid biotin attachment to the cladding, the amino-silanized fibers were polished for 10 seconds with 0.3 $\mu$m lapping film, which removed the top amino-silanized cladding layer from the fiber array *(see* Figure 1c). After polishing, NHS-biotin was attached to the amino groups on the well surfaces *(see* Figure 1d).

### Methods

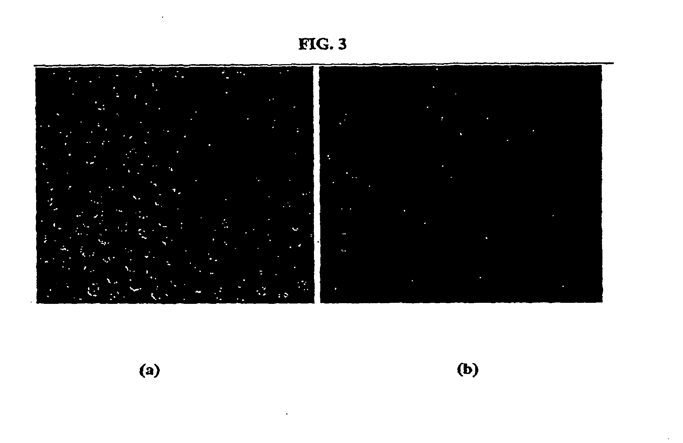

First, the effectiveness of the capture component was tested. To test the effectiveness of the biotinylation of the substrate, streptavidin Alexa Fluor 568® was attached directly to the biotin groups on the surfaces of both a polished and an unpolished fiber, followed by image acquisition of the modified surface *(see* FIG. 3). FIG. 3 shows Streptavidin Alexa Fluor 568® binding to (a) an unpolished biotin modified fiber optic array, and (b) a polished biotin modified fiber optic array. As seen in image (a), streptavidin binding occurred on all surfaces, in comparison to image (b), where binding occurred only on the surfaces of the microwell reactors. Thus, the unpolished fiber shows dye over the entire array including the cladding surface, while the polished fiber shows dye localized only on the well surfaces.

Subsequent to array modification, the biotinylated fiber arrays were incubated for 1 hour at room temperature

in 150 $\mu$L PBS buffer containing varying amounts of S$\beta$G. The concentration of the S$\beta$G was chosen so that during the incubation time, statistically either one molecule or no molecules would bind to each well. The arrays were then washed repeatedly in PBS buffer, to ensure that unbound target was removed.

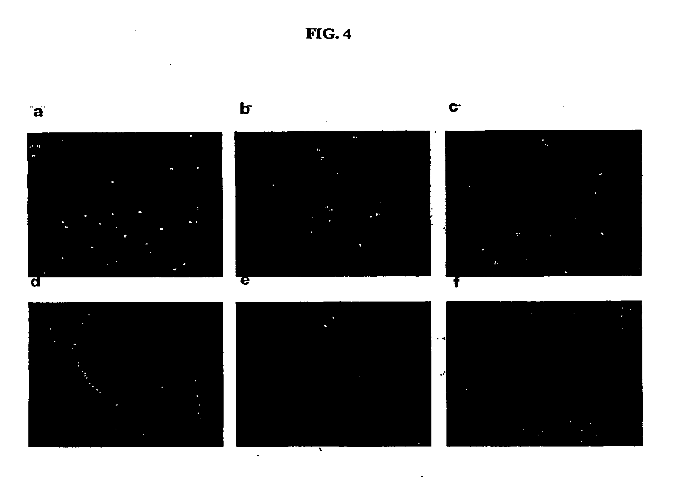

For a binary readout of S$\beta$G binding, the fiber array was loaded and secured on an. upright microscope system equipped with a mechanical platform. A solution of $\beta$-galactosidase substrate, resorufin-$\beta$-D-galactopyranoside (RDG), was introduced to the distal end of the fiber containing the reaction vessels, and subsequently sealed. The substrate was sealed using a 0.01-inch thick silicone elastomer gasket sandwiched between a microscope slide and the fiber array by means of a mechanical platform located beneath the microscope stage. This platform applied a uniform pressure to the gasket material, across the entire bundle, sealing off each reaction chamber and enabling well to well interrogation of enzyme activity. $\beta$-galactosidase hydrolyzes RDG to form resorufin, which builds up to a locally high concentration in each sealed reaction vessel, generating a detectable fluorescent signal (Figure 4).

FIG. 4 depicts a portion of the fiber array for each experiment. Each of the experiments tested a different sample having a different concentration of S$\beta$G. The concentrations for each experiment were as follows: (a) 128 amol, (b) 51 amol, (c) 25 amol, (d) 7.5 amol, and (e) 2.6 amol. FIG. 4(f) depicts the control.

Analysis of over 5000 reaction vessels for each experiment allowed for a correlation between the percentage of reaction vessels that captured an enzyme molecule and the amount of enzyme present in the interrogated sample. The variation seen in the intensity differences from active well to active well is most likely a result of molecule-to-molecule variation in catalytic activity, in combination with surface effects, which may modulate the relative activities of enzyme molecules based on their orientation to the reaction chamber surface.

Two control experiments were also conducted to ensure that the binding of enzyme to the surface of the reactors was based exclusively on the biotin-streptavidin interaction, and not on non-specific binding to the glass surface. One control experiment consisted of an etched, unmodified fiber incubated with the most concentrated S$\beta$G target solution (128 amol in 150 $\mu$L). The second control experiment was performed using the modified fiber incubated in a solution of $\beta$-galactosidase lacking streptavidin (128 amol in 150 $\mu$L). Both control experiments generated a negligible active well percentage (less than 0.06%, versus 0.2% for the 2.6 amol experiment discussed below).

Results

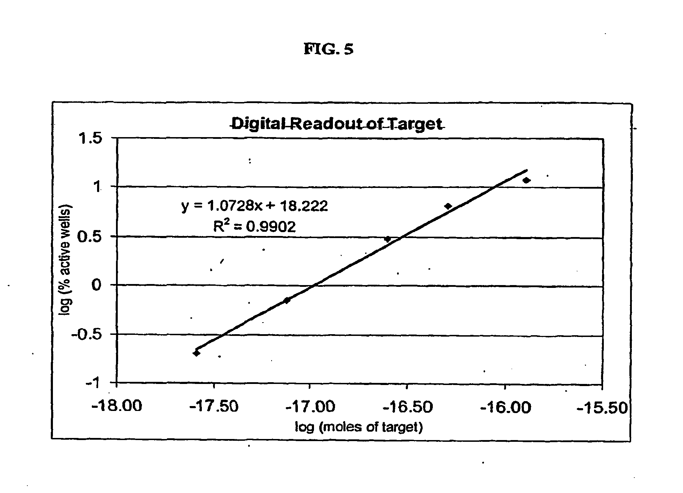

FIG. 5 depicts a log-log plot of the moles of target present in a sample with the resulting percentage of active reaction vessels. The linear relationship between the percentage of active reaction vessels and the moles of target in the log-log plot shown in FIG. 5 suggests that a binary readout detection method can be used for the detection of real targets such as DNA and antigens. This method permits rapid analysis and accurate concentration information via digital readout, while maintaining a straightforward assay procedure.

It is also interesting to note that the lowest limit of detection (LOD) for binding streptavidin-$\beta$-galactosidase (S$\beta$G) to a biotinylated femtoliter array in this example was 2.6 amoles (150 $\mu$L of 17 fM solution) using a target incubation time of 1 hour.

**Example 2**

In this example, single molecules of $\beta$-galactosidase were monitored using a 1 mm diameter fiber optic bundle with over $2.0 \times 10^5$ individually sealed, femtoliter microwell reactors. By observing the buildup of fluorescent products from single enzyme molecule catalysis over the array of reaction vessels and by applying a Poisson statistical analysis, a digital concentration readout was obtained.

Materials

1mm bundled 4.5 $\mu$m optical fibers were purchased from Illumina (San Diego, CA). $\beta$-galactosidase and Ru(bpy)$_3$Cl$_2$ was obtained from Sigma-Aldrich (St. Louis, MO). Resorufin-D-$\beta$-galactopyranoside was purchased from Molecular Probes (Eugene, OR). 0.01-inch non-reinforced gloss silicone sheeting material was purchased from Specialty Manufacturing Inc. (Saginaw, MI). All other chemicals used were of reagent grade and obtained from Sigma-Aldrich (St. Louis, MO).

A custom-built, upright epifluorescence imaging system acquired all fluorescence images using a mercury light source, excitation and emission filter wheels, microscope objectives, and a CCD camera (QE, Sensicam). Filter wheels and shutters were computer controlled and analysis was performed with IPlab software (Scanalytics, Fairfax, VA). The system was equipped with a fastening device to fix the fiber optic array onto the system through the entire experiment. A mechanical platform beneath the stage was used to house the silicone-sealing layer, which was subsequently brought into contact with the distal end of the fiber array, sealing off each reaction vessel. All measurements were performed with ferntowell arrays at the distal end of the optical fiber bundle.

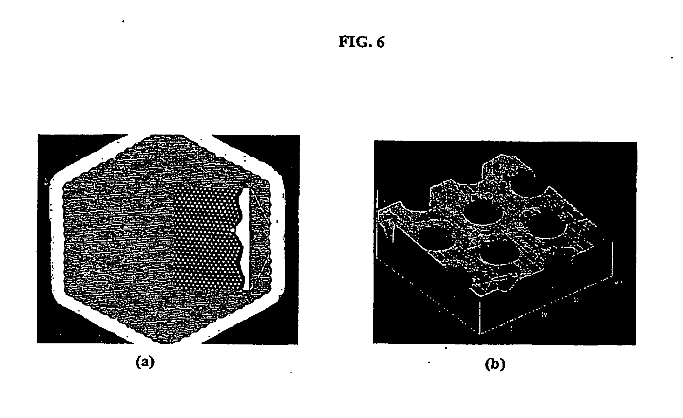

. Optical fiber bundles containing approximately 2.4 x $10^5$ individual 4.5 $\mu$m diameter optical fibers were used as the substrate for fabricating femtoliter reaction vessel arrays. The well volume can be precisely controlled, as etch depth varies with etch time and etchant concentration. The optical fibers used in these experiments were etched to a depth of approximately 2.9 $\mu$m, yielding a 46 fL well volume. FIG. 6 depicts images of the etched surface of the fiber optic bundles. More specifically, FIG. 6a depicts the entire fiber array and close-up microscope images of the fiber bundle, emphasizing the regularity of both the array and each individual optical fiber. Further, FIG. 6b is an AFM image of a portion of the etched surface, showing wells created from the etching process.

Methods

*Assay.* For the β-galactosidase assay, the substrate used was resorufin-β-D-galactopyranoside. After the individual wells in the array were sealed in the presence of enzyme and substrate, the fluorescence intensity was monitored across the array of vessels for the enzymatic product, resorufin (ex 558 nm / em 573 nm). A 100 $\mu$M solution of resorufin-D-β-galactopyranoside (RDG) was prepared in 100mM Tris buffer pH 8.0 containing 2.0 mM KCl and 0.1 mM MgCl$_2$. All enzyme solutions were prepared from previously aliquoted and frozen stock samples in the same reaction buffer. Just prior to experimentation, the two samples were centrifuged for 2 min at 7000 RPM to remove any particulate material that could interfere with the mechanics of the silicone seal. Approximately 1 cm$^2$ of silicone and a microscope slide were cleaned with absolute ethanol. The silicone sheeting was placed on the surface of the glass, to which it adhered. Subsequently, 75 $\mu$L volumes of enzyme and RDG solutions were mixed on the silicone gasket using a pipette. The gasket was mechanically raised towards the distal end of the fiber bundle until it experienced resistance, suggesting that a seal was formed. An initial fluorescence image was acquired, followed by periodic image acquisition for approximately 2 hr.

*Sealing component.* To seal the femtoliter array, a 0.01-inch thick silicone elastomer gasket was sandwiched between a microscope slide and the fiber array using a mechanical platform. This platform applied uniform pressure to the gasket material, across the entire bundle, sealing off each microwell to create the reaction vessels.

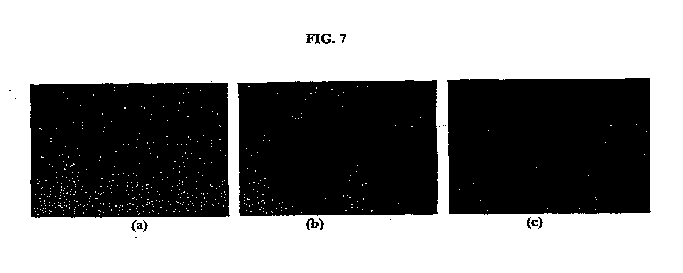

The silicone/glass seal used to create and isolate the femtoliter containers was inspected for its sealing ability by performing a photobleaching experiment *(see* Fig. 7). FIG. 7 depicts enclosure of a solution into the microchambers and evaluation of the silicone seal for integrity. FIG. 7a depicts a solution of Ru(bpy)$_3$Cl$_2$ enclosed into the array of chambers as observed by the red fluorescence across the array. FIG. 7b depicts a small octagonal portion of the fiber bundle that was photobleached via UV light. FIG. 7c depicts the array 60 minutes later. As shown in the figure, diffusion of Ru(bpy)$_3$Cl$_2$ from one well to another as a result of an imperfect silicone seal would display increased fluorescence intensity in photobleached wells and was not observed. This experiment substantiated the integrity of the seal for its ability to successfully isolate the array of vessels. Enzyme molecule denaturation on the glass surface was prevented by blocking with a BSA blocking buffer. Enzyme to vessel ratios used ranged from 1:5, down to 1:500, achieving accurate detection over two orders of magnitude.

*Photobleaching Experiment.* A solution of 1mM Ru(bpy)$_3$Cl$_2$ in DI water was used for the photobleaching experiments. A piece of silicone, approximately 1 cm$^2$, and a microscope slide were cleaned with absolute ethanol using lint-free swabs. The silicone sheeting was placed on the surface of the glass, to which it adhered. 50 $\mu$L of the Ru(bpy)$_3$Cl$_2$ solution was placed on the silicone, and subsequently brought into contact with the fiber bundle, to enclose the solution in the individual vessels. Using a field stop on the imaging system, UV light was used to illuminate a small portion of the array for 10 minutes, photobleaching the Ru(bpy)$_3$Cl$_2$. The field stop was then opened, and an image was acquired, displaying the difference in fluorescence. The array was then allowed to rest with the seal maintained. A final image was taken after 60 minutes, confirming the integrity of the seal.

As discussed above, the number and volume of reaction vessels employed govern the dynamic range of concentrations that can be determined by this technique. The reaction vessel volumes employed in this example were 46 fL (vide infra); therefore, it was calculated that a solution of 3.6 x $10^{-11}$ M β-galactosidase will yield, on average, one enzyme molecule per vessel. As also discussed above, if the concentrations used are much less than 3.6 x $10^{-11}$ M, the expected average becomes exceptionally low, the distribution is narrowed, and the probability of observing anything other than 0 or 1 events per trial is improbable in all experimental cases. At these low concentrations, the relationship between the percentage of active reaction vessels and the bulk enzyme concentration is approximately linear. After waiting for sufficient time to allow enzyme catalysis to occur, individual vessels were interrogated for an on/off response, correlating to each vessel either possessing or lacking enzymatic activity.

The substrate resorufin-D-β-galactopyranoside (RDG) was used as the substrate for experiments, which was sealed into all the vessels, along with the trapped enzyme molecules, using a silicone gasket material and mechanical arm. The expected percentages of active wells were calculated for each concentration used by applying the Poisson distribution statistics.

Results

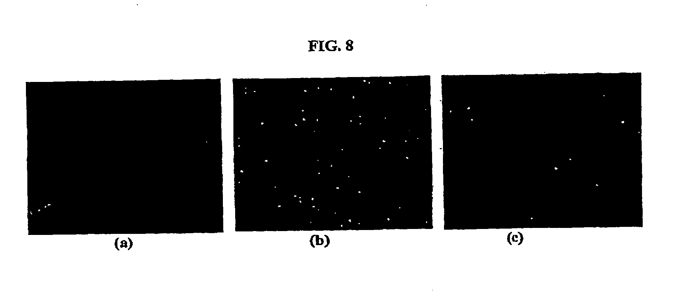

As shown in FIG. 8, for the β-galactosidase assay, different bulk solution enzyme concentrations correspond to different ratios of enzyme to vessel volume, resulting in variation in the percentage of vessels that contain an enzyme molecule. Figure 8 depicts the detection of the activity of single molecules of β-galactosidase. FIG. 8a is a background image of a portion of the array, while FIG. 8b depicts an image taken of a portion of a 1:5 enzyme to vessel assay, and FIG. 8c shows a 1:80 enzyme to vessel assay.

Table 1 is a comparison of each experimental result with the percentage of occupied vessels calculated from the Poisson distribution. As shown by the data in the table, the array measurements successfully correlated with the number of single enzyme β-galactosidase molecules over the entire range of interrogated concentrations. There is minor disparity in the observed signals as a result of molecule-to-molecule variation in catalytic activity. This result is most likely due to the inherent stochastic nature of enzymes, in addition to surface effects, resulting in modulation of enzyme activity.

Table 1. Digital readout from the arrays. The actual percentage of chambers exhibiting activity, in comparison to the expected percentage calculated from the Poisson distribution, are listed for the various concentrations analyzed.

| Digital Readout of Enzyme Concentrations | | | |
|---|---|---|---|
| **Enzyme to well ratio** | **Concentration** | **Poisson % of active wells** | **Actual % active** |
| 1:5 | 7.20E-12 | 18.2 | 14.9 |
| 1:10 | 3.60E-12 | 9.5 | 11.5 |
| 1:20 | 1.80E-12 | 4.9 | 5.6 |
| 1:40 | 9.00E-13 | 2.5 | 3.5 |
| 1:80 | 4.50E-13 | 1.2 | 1.5 |
| 1:100 | 3.60E-13 | 1.0 | 1.1 |
| 1:200 | 1.80E-13 | 0.5 | 0.3 |
| 1:500 | 7.20E-14 | 0.2 | 0.1 |

The variation between the calculated and experimental results can be attributed to the intrinsic variability associated with the probability distribution, as well as experimental error in the preparation of enzyme solutions.

**Example 3**

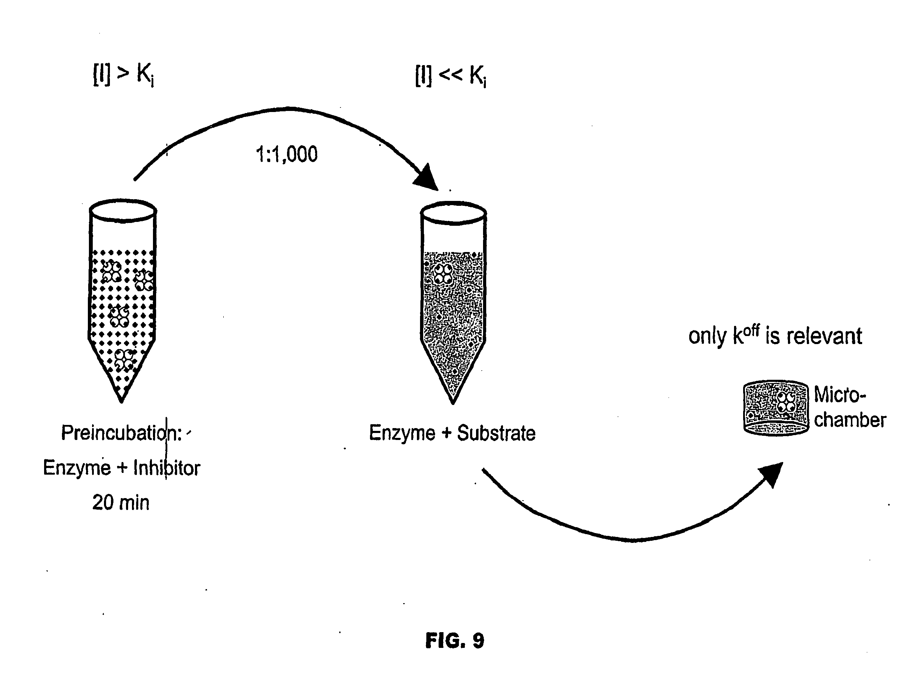

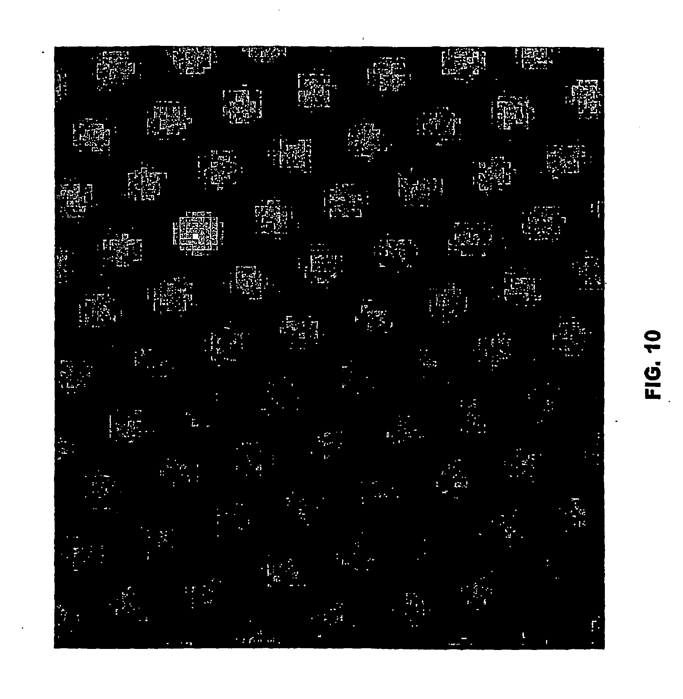

In this example, beta-galactosidase was studied in the presence of a competitive inhibitor, D-galactal (a transition state analog of D-galactose) as a model enzyme-inhibitor-pair. The scope of this application shall, however, not be limited to this particular enzyme-inhibitor-pair. Beta-galactosidase is a tetramer and D-galactal is known to exhibit relatively slow binding and release. Figure 9 shows one embodiment of the scheme for measuring single molecule inhibition, as well as detection of inhibitor release. In a first step, the enzyme and inhibitor are preincubated in an aqueous buffer system such as a phosphate buffer (in this particular embodiment PBS/$MgCl_2$-buffer: 2.7 mM KCl, $KH_2PO_4$, 1.5 mM $KH_2PO_4$, 136 mM NaCl, 8.1 mM $Na_2HPO_4$, 1 mM $MgCl_2$, pH 7.3) together in bulk, such that the inhibitor concentration ([inhibitor]) is higher than the dissociation constant for inhibitor binding ($K_i$) and all of the active sites of the enzyme are likely to be blocked or occupied. As $K_i$ differs for different enzyme/inhibitor pairs, the concentration of inhibitor must be adjusted. With this particular enzyme/inhibitor pair used in this example a variety of inhibitor starting concentrations can be employed, ranging between 100 mM and 20 μM. In this embodiment, [inhibitor] was 100 μM (~ 7 x $K_i$ (14 μM)). In a second step, one or more substrates (e.g. rsesorufin-beta-galactopyranoside RGP) in an aqueous buffer system (in this particular embodiment: PBS/$MgCl_2$-buffer) are added, preferably in a high volume excess (between 10-fold and 100,000-fold). In this particular embodiment a 1000-fold volume excess was used, thereby diluting the inhibitor and the enzyme 1000-fold. More components (in this particular embodiment 1% (w/v) bovine serum albumin (BSA) to avoid unspecific binding to the surface of the arrays of sites) may be added to the buffer system. In a third step, the bulk solution is added to the array of sites, each site having a defined volume (between 10 attoliters and 50 picoliters, more preferably femtoliters, in this embodiment: 46 femtoliters). At this point, the inhibitor is not in excess any more; indeed, [inhibitor] << $K_i$ (it is preferred that less than 100,000 molecules of inhibitor, and preferably less than 10,000 molecules of inhibitor, and still more preferably less than 3,000 molecules of inhibitor, are remaining in the solution). The enzyme concentration (1.8 nM) is adjusted so that upon 1,000-fold dilution in step 2 there will be only a single enzyme in every array site in some sites and no enzyme in others. Figure 9 shows schematically the situation where one of the active sites of the tetramer is unblocked and active. This active site would be capable of interacting with a substrate that includes a reporter-moiety. This domain may create a chromogenic or a fluorogenic signal upon enzymatic cleavage but is not limited to these. Preferably a fluorogenic signal is generated, and more preferably RGP is used as the fluorogenic substrate. In this way the site in the array with such activity can be readily detected (Figure 10).

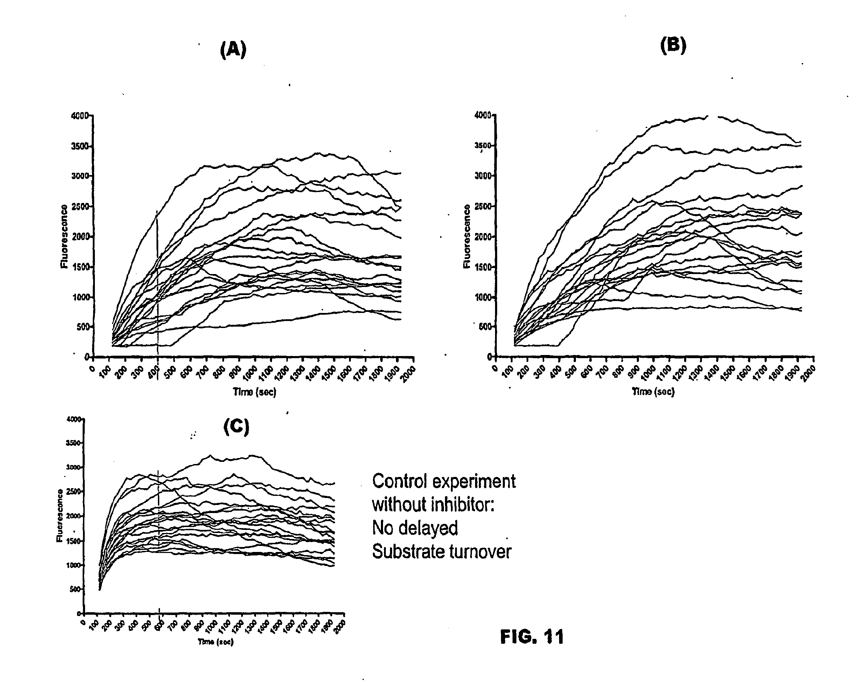

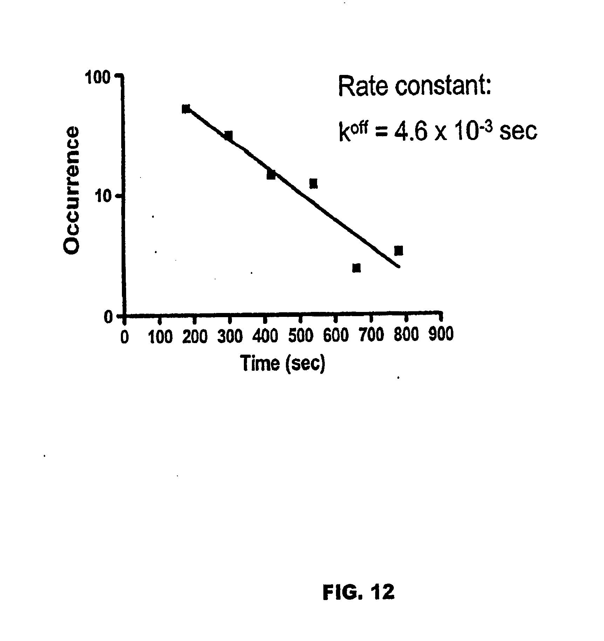

In one embodiment, the present invention contemplates determining substrate turnover at time points (e.g. intervals of seconds, tens of seconds, 100s of seconds, minutes, tens of minutes, etc.). Figure 11 shows that when assayed according to the scheme of Figures 9 and 10, the presence of an inhibitor delays substrate turnover, as compared to a control (lower left panel) where no inhibitor is present. When the frequency distribution of substrate turnover onset times is plotted (Figure 12), the $K_{off}$ rate constant can be calculated: $4.6 \times 10^{-3}$ sec. This agrees well with published values.

Interestingly, the data suggests that release of inhibitor at each of the four sites of the tetramer is not independent. That is to say, if there are 4 independent catalytic sites of a tetramer and $k^{off}$ is a first order rate constant then $k^{off}$ of an enzyme ensemble is the same as $k^{off}$ of each protomer. But as each single enzyme contains 4 protomers there are 4 possibilities to release a first inhibitor. Therefore: $k^{off}_1 = 4 \times k^{off}(= 1.8 \times 10\text{-}2 \text{ sec-1})$. The data suggests that the inhibitor release from the protomers is non-independent.

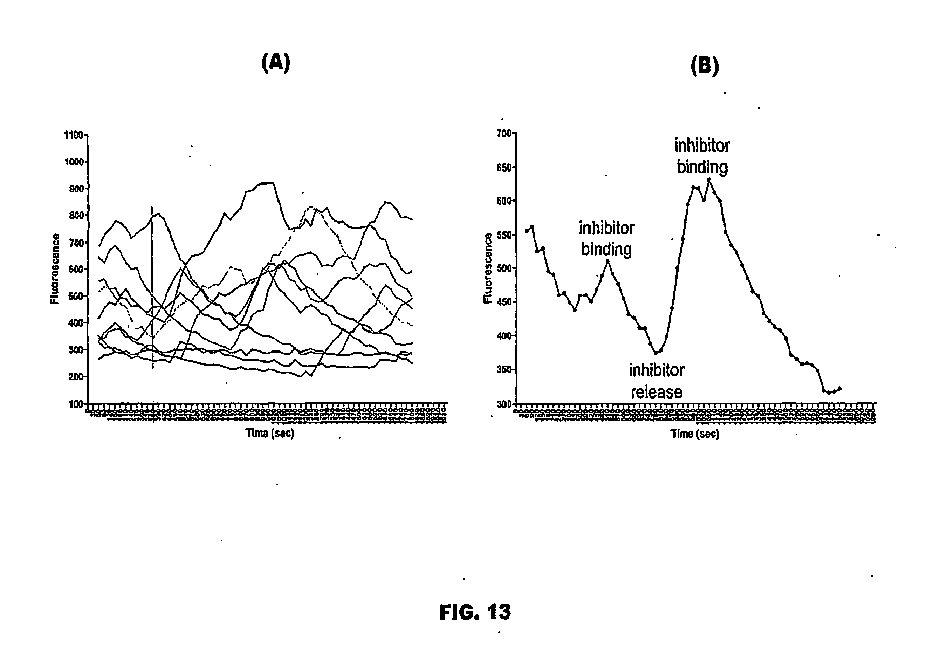

In a second embodiment without preincubation the inhibitor is given to the enzyme in an aqueous buffer system such as a phosphate buffer (in this particular embodiment PBS/MgCl$_2$-buffer. More components (in this particular embodiment 1% (w/v) (BSA) to avoid unspecific binding to the surface of the arrays of sites) may be added to the buffer system. The [inhibitor] is chosen in the same range as Ki (14 $\mu$M), preferably between 100 mM and 1 $\mu$M, in this embodiment 20 $\mu$M. At this inhibitor concentration the inhibitor-bound enzyme is in equilibrium with the inhibitor-free enzyme. The bulk solution is added to the array of sites, each site having a defined volume (between 10 attoliters and 50 picoliters, more preferably femtoliters, in this embodiment: 46 femtoliters). The enzyme concentration (1.8 pM) is chosen to yield only a single enzyme in some sites and no enzyme in other sites. When fluorescence is monitored over time, one can see binding, release, and subsequent binding (Figure 13). Figure 13A (left panel) represents the raw data from various time traces. Figure 13B (the right panel) simply shows one of the time traces of the left panel, where for the sake of clarity, the y-axis is scaled differently. Figure 13B shows that the signal increases after the inhibitor has been released from the enzyme and it decreases after the inhibitor has been bound. One should expect that the fluorescence signal remains constant over time if the inhibitor is bound to the enzyme as there is no enzyme activity. But this is not the case because there is a substantial amount of photobleaching that depletes the fluorophor continuously. This creates a dynamic with the net results (over time) shown in Figure 13.

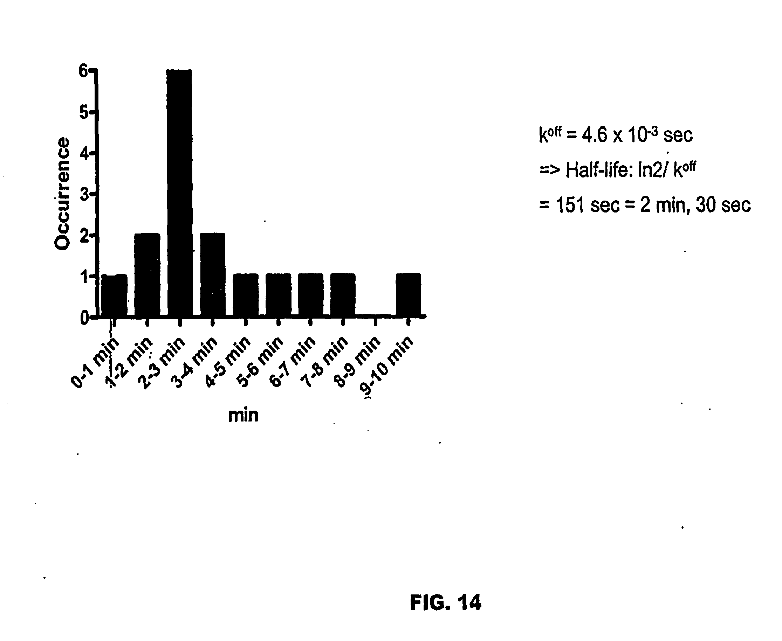

Figure 14 shows a histogram of off-times (the time how long the inhibitor stays on the enzyme before it is released. This permits a calculation of the half-life (the half-life of a quantity subject to exponential decay is the time required for the quantity to decay to half of its initial value) which is experimentally determined to be approximately two minutes and thirty seconds. This example demonstrates that a system that measures single enzymes may reveal more kinetic data about how the enzyme subunits interact than were previously available from ensemble reactions.

**Example 4**

In one embodiment, nanoparticles are utilized for the digital readout of target concentrations. Nanoparticles have several advantages over an enzyme digital readout. With an enzyme approach, as concentrations increase, the linearity of the digital readout, which follows a Poisson distribution, is lost. Using nanoparticles functionalized with fluorescent species, the present invention contemplates in one embodiment to be able to perform a digital readout as well as the traditional bulk intensity readout, which is used extensively in our laboratory. As the linearity of the digital signal is lost, the analysis can be shifted from a digital readout to a traditional intensity readout using the nanoparticles. The signal can then be read as an average intensity of each site in the array (e.g. average well intensity) correlating to concentration, and will significantly increase the range of concentrations that can be read with this system. In some embodiments employing nanoparticles, the array is not sealed.

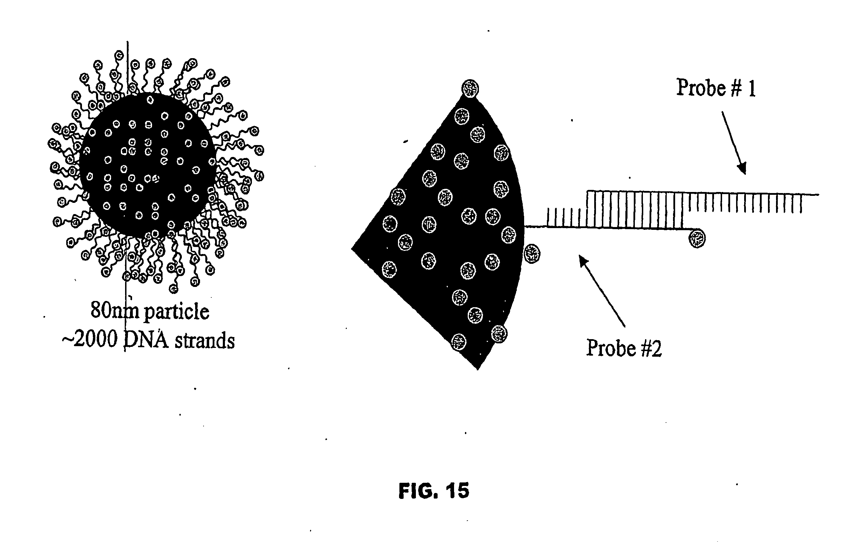

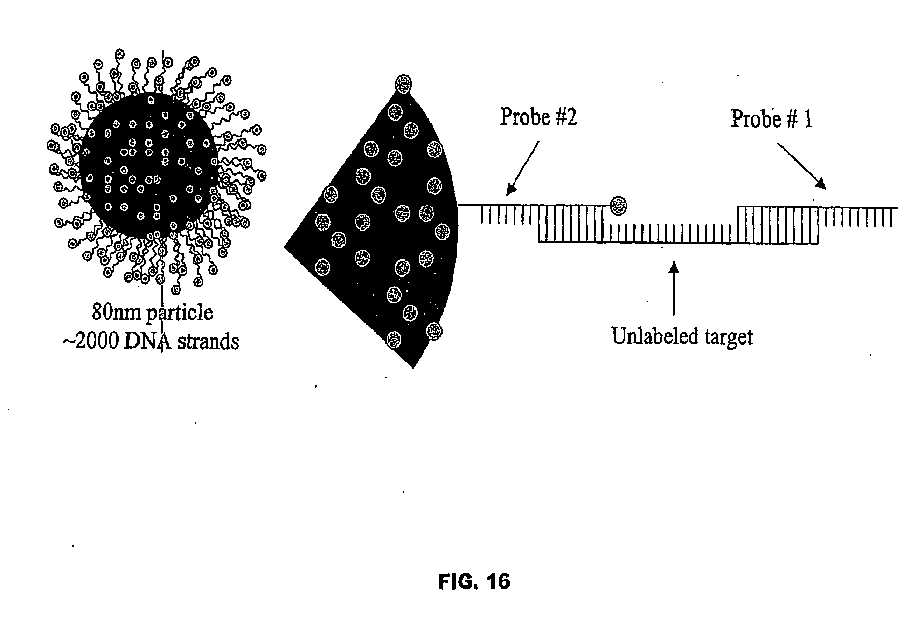

Nanoparticles such as gold particles (e.g. between 1 and 500 nanometer, and more preferably between 50 and 250 nanometer, and still more preferably between 80-150 nanometer gold particles) may be attached to the target analyte or a ligand that will bind a target analyte. A variety of embodiments are contemplated, including but not limited to embodiments where the target is attached to the gold particle and introduced into an array, said array comprising a binding partner (e.g. immobilized ligand). In another embodiment, the target analyte may be attached or otherwise immobilized on the array and the gold particle-binding partner conjugate can be introduced to the array to bind the target analyte. In another embodiment, a first binding ligand may immobilize the target, and the gold-particle-second binding ligand may be introduced into the array to bind the target. These embodiments are schematically illustrated in Figures 15 and 16, where the target is nucleic acid and the binding partners are complementary probes. In Figure 15, either probe # 1 or probe # 2 could be replaced with target, if desired. In Figure 16 shows a two-step sandwich format, where the target can be unlabeled and first incubated with probe #1; thereafter, the nanoparticle with probe #2 is introduced. The target can be viewed to operate as a linker in this embodiment.

In the present example, the target analyte is nucleic acid, and the gold particles are labeled with a plurality (e.g. approximately 2000-8000 strands) of fluorescent DNA; this DNA-gold particle conjugate will be used to generate signal. Because of the high number of fluorescent DNA strands attached to each nanoparticle, a single particle is sufficient for a signal spike over background in a single site (e.g. microwell) of the array (e.g. the fiber optic array described above). The fluorescence intensity deviation over a population of nanoparticles is very small in comparison to enzymes, whose catalytic rate is highly fluctuating. Because of this high fluctuation rate, only a yes or no response can be deciphered, limiting the range of detection for enzymes to approximately two orders of magnitude. Using nanoparticles that have equivalent signal intensities will allow for a more controlled readout of 0 or 1 as carried out with the enzymes, along with readouts of 2, 3, 4, and so on. The ability to readout discreet intensity levels will enable a superior range of detection, well beyond two orders of magnitude. Analyte-functionalized gold nanoparticles (e.g. DNA-gold particle conjugates or other biomolecule-gold particle conjugates) will improve the limit of detection, as they consistently show extremely low levels of non-specific and cross-reactive binding in analyte (e.g. DNA) detection schemes.

A. Preparation of Gold Particles

Gold particles can be prepared a number of ways (Analytical Chemistry, 2000, 72, 5535-5541; Science, 2002, 296, 1836-1838; Science, 2002 295, 1503-1506; J. Am. Chem. Soc. 2004, 126, 5932-5933). In one case, gold colloids (13 nm diameter) are prepared by reduction of $HAuCl_4$ with citrate as described in Frens, Nature Phys. Sci., 241, 20 (1973) and Grabar, Anal Chem., 67, 735 (1995). Briefly, all glassware is cleaned in aqua regia (3 parts HCl, 1 part $HNO_3$), rinsed with Nanopure $H_2O$, then oven dried prior to use. $HAuCl_4$ and sodium citrate are purchased from Aldrich Chemical Company. Aqueous $HAuCl_4$ (1 mM, 500 mL) is brought to reflux while stirring. Then, 38.8 mM sodium citrate (50 mL) is added quickly. The solution color will change from pale yellow to burgundy, and refluxing should continue for 15 min. After cooling to room temperature, the red solution can be filtered through a Micron Separations Inc. 1 micron filter. Au colloids are readily characterized by UV-vis spectroscopy using a Hewlett Packard 8452A diode array spectrophotometer and by Transmission Electron Microscopy (TEM) using a Hitachi 8100 transmission electron microscope. Gold particles with diameters of 13 nm will produce a visible color change when aggregated with target and probe oligonucleotide sequences in the 10 to 35 nucleotide range.

B. Fiber Modifications

In this example, a fiber array is employed. The polished fiber array is etched for 115 seconds in 0.025 M HCl giving 46 fL wells. For cleaning, the etched fibers is incubated in boiling Nanopure water for 10 minutes.
The surface of the etched fiber can be modified in a number ways. In this example, an amine modification of the fiber is employed. First, a silanization solution is prepared, i.e. a 1% solution of DETA (trimethoxysilylpropyldiethylenetriamine) in a 95:5 solution of ethanol:nanopure water. The etched fiber was incubated in the silanization solution for 20 minutes at room temperature, followed by washing with nanopure water and drying under argon. The fiber was then cured at 120°C for 15 minutes. At this point, the fiber is now modified with amine groups.
A variety of functional groups can be reacted with the amine groups. In this example, the process involved maleimide modification of the fiber. 3.55 mg of SMPB (Succinimidyl 4-[p-maleimidophenyl]butyrate) was dissolved in 1 mL DMSO to create a stock solution. A cross-linking solution was prepared: 100 $\mu$L of this stock solution was added to 900 $\mu$L of a 90:10 solution of ethanol:DMSO. The silanized fibers are incubated in the crosslinking solution for 2 hours at room temperature. The array is then washed with an 80:20 solution of ethanol:DMSO and dried under argon. At this point, The fiber is now modified with maleimide groups.

C. DNA Deprotection

2 nanomoles of thiol modified (protected) DNA is reconstituted in 110 $\mu$L PBS pH 7.4. 10 $\mu$L DTT (dithiothreitol) stock (1.0 M) is added to the protected DNA solution and incubated for 2 hours at room temperature. A NAP-5 column (GE Healthcare) was used to purify the deprotected DNA. The column was equilibrated with Nanopure water.
The 120 $\mu$L solution of deprotected thiol DNA was added to the column and allowed to completely enter the gel. 380 $\mu$L PBS 7.4 water was added to the column and allowed to completely enter the gel. A collection tube was placed under the column and the thiol DNA was eluted by adding 1.0 mL PBS 7.4 water to the column. The first 400 $\mu$L was discarded, and DNA was collected from 400 $\mu$L to 1 mL, as described in the NAP-5 column protocol (provided by the manufacturer).

D. DNA Attachment

A ligand (in this case, a probe) specific for the target analyte (in this case, nucleic acid) can be attached directly to the sites of the array or to the nanoparticle (e.g. gold particle) and then the conjugate (DNA-gold nanoparticle) can introduced into the array. In this example, the maleimide-treated fibers were incubated in the thiol deprotected DNA solution (1 μM) for 2 hours at room temperature. The fibers were rinsed with PBS 7.4 to remove unbound DNA and the array was swabbed to remove DNA probes that were bound to the cladding material. Gold nanoparticles (in this case 80 nm) were then incubated with the fiber and preliminary experiments show successful binding (Figure 16).

**Example 5**

This example describes the immobilization of synthetic single-stranded DNA on semiconductor nanoparticle quantum dots (QDs). Native CdSe/ZnS core/shell QDs (-4 nm) are soluble only in organic media, making direct reaction with alkylthiol-terminated single-stranded DNA difficult. This problem is circumvented according to Mirkin et al. by first capping the QDs with 3-mercaptopropionic acid. The carboxylic acid group is then deprotonated with 4-(dimethylamino)pyridine, rendering the particles water soluble, and facilitating reaction of the QDs with either 3'-propylthiol- or 5'-hexylthiol-modified oligonucleotide sequences. After DNA modification, the particles are separated from unreacted DNA by dialysis. A "linker" DNA strand can then be hybridized to surface-bound sequences, generating extended assemblies of nanoparticles. The QD assemblies are readily characterized by TEM, UV/Visible spectroscopy, and fluorescence spectroscopy. Reportedly, they can be reversibly assembled by controlling the temperature of the solution. As a result, QD assemblies and composite aggregates can be formed between QDs and nanoparticles (e.g. gold nanoparticles of about. 13 nm).

**Bibliography**

Sano, T.; Smith, C. L.; Cantor, C. R. Science 1992, 258, 120-122.

Nam, J. M.; Thaxton, C. S.; Mirkin, C. A. Science 2003, 301, 1884-1886.

Niemeyer, C. M.; Adler, M.; Pignataro, B.; Lenhert, S.; Gao, S.; Chi, L. F.; Fuchs, H.; Blohm, D. Nucleic Acids Research 1999, 27, 4553-4561.

Zhou, H.; Fisher, R J.; Papas, T. S. Nucleic Acids Research 1993, 21, 6038-6039.

Niemeyer, C. M.; Adler, M.; Wacker, R. Trends in Biotechnology 2005, 23, 208-216.

Whitesides, G. M. Nature Biotechnology 2003, 21, 1161-1165.

Rondelez, Y.; Tresset, G.; Tabata, K. V.; Arata, H.; Fujita, H.; Takeuchi, S.; Noji, H. Nature Biotechnology 2005, -23, 361-365.

Nakano, M.; Komatsu, J.; Matsuura, S.; Takashima, K.; Katsura, S.; Mizuno, A. Journal of Biotechnology 2003, 102, 117-124.

Nagai, H.; Murakami, Y.; Yokoyama, K.; Tamiya, E. Biosensors and Bioelectronics 2001,16, 1015-1019.

Lipman, A. E.; Shuler, B.; Bakajin, O.; Eaton, W. A. Science 2003, 301, 1233-1235.

Chiu, D. T.; Wilson, C. F.; Ryttsen, F.; Stromberg, A.; Farre, C.; Karlsson, A.; Nordholm, S.; Graggar, A.; Modi, B. P.; Moscho, A.; Garza-Lopez, R. A.; Orwar, O.; Zare, R. N. Science 1999, 283, 1892-1895.

Rissin, D. M.; Walt, D. R. Journal of the American Chemical Society **submitted.**

Pantano, P.; Walt, D. R. Chemistry of Materials 1996, 8, 2832-2835.

Monk, D. J.; Ueberfeld, J.; Walt, D. R. Journal of Materials Chemistry 2005, 15, 4361-4366.

Song, L. N.; Ahn, S.; Walt, D. R. Emerging Infectious Diseases 2005, 11, 1629-1632.

Lee, J. Y.; Li, H. W.; Yeung, E. S. Journal of Chromatography A 2004, 1053, 173-179.

Xue, Q. F.; Yeung, E. S. Nature 1995, 373, 681-683.

Foquet, M.; Korlach, J.; Zipfel, W. R.; Webb, W. W.; Craighead, H. G. Analytical Chemistry 2004, 76, 1618-1626.

Gratzl, M.; Lu, H.; Matsimoto, T.; Yi, C.; Bright, G. R. Analytical Chemistry 1999, 71, 2751-2756.

Stamou, D.; Duschl, C.; Delamarche, B.; Vogel, H. Angewandte Chemie-International Edition 2003, 42, 5580-5583.

Gosalia, D. N.; Diamond, S. L. Proceeding of the National Academy of Sciences USA 2003, 100, 8721-8726.

Lu, H. P.; Xun, L. Y.; Xie, X. S. Science 1998, 282, 1877-1882.

Taylor, J. R. An Introduction to Error Analysis; Second Addition ed.; University Science Books: Sausalito, CA, 1997.

Wheeler, A. R.; Throndset, W. R.; Whelan, R. J.; Leach, A. M.; Zare, R. N.; Liao, Y. H.; Farrell, K.; Manger, L D.; Daridon, A. Analytical Chemistry 2003, 75, 3581-3586.

Angenendt P.; Lehrach H.; Kreutzberger J.; Glökler J. Proteomics, 2005, 5, 420-425.

**Claims**

1.  A method of measuring the concentration of a target analyte in a sample, comprising:

    (a) providing a sample and an array of capture components, said sample comprising molecules of target analyte, said array comprising at least 1000 sites, each site having a defined volume between 10 attoliters and 50 picoliters;
    (b) contacting said array with said sample so that sites contain target analyte bound to capture components such that the ratio of molecules of target analyte to sites of said array is less than 1:5 which results in sites with single analyte molecules; and
    (c) determining the percentage of sites of said array which contain a target analyte molecule to provide a measure of the target analyte concentration.

2.  The method of claim 1, wherein said molecules of target analyte comprise optionally labelled biomolecules.

3.  The method of claim 2, wherein said biomolecules are selected from the group consisting of proteins, nucleic acids, lipids, carbohydrates, hormones, cytokines, cellular antigens, and receptors.

4.  The method of claim 3, wherein said biomolecules are receptors selected from the group consisting of neural receptors, hormonal receptors, and nutrient receptors.

5.  The method of claim 1, wherein said target analyte is selected from the group consisting of environmental pollutants, pesticides, insecticides, toxins and drugs.

6.  The method of claim 1, wherein said ratio is (i) between 1:5 and 1:500; or (ii) 1:10.

7.  The method of claim 1, further comprising, after the contacting step, sealing the array such that the contents of each site cannot escape said site.

8.  The method of claim 1, wherein said defined volume of each site ranges from 30 femtoliters to 60 femtoliters.

9.  The method of claim 1, wherein said array comprises (i) between 20,000 and 10,000,000 sites; or (ii) between 20,000 and 30,000 sites; or (iii) between 100,000 and 10,000,000 sites, or (iv) between 10,000 and 50,000 sites.

10. The method of claim 1, wherein each site of said array comprises a capture component.

11. The method of claim 10, wherein said molecules of target analyte are selected from the group consisting of enzymes, DNA, or antibodies.

12. A method of claim 1, wherein the array comprises a plurality of microwells formed in one end of a fiber optic bundle.

13. The method of claim 1, wherein said determining comprises detecting a change in an optical property at said sites as an indication of the presence of said target analyte molecule.

**Patentansprüche**

1.  Verfahren zur Messen der Konzentration eines Zielanalyten in einer Probe, umfassend:

    (a) Bereitstellen einer Probe und eines Arrays aus Einfang-Komponenten (capture components), wobei die Probe Moleküle des Zielanalyten umfasst, das Array mindestens 1.000 Stellen (sites) umfasst, jede Stelle ein definiertes Volumen zwischen 10 Attolitern und 50 Pikolitern hat;
    (b) in Kontakt bringen des Arrays mit der Probe, so dass Stellen den an die Einfang-Komponenten gebundenen Zielanalyten enthalten und das Verhältnis vom Molekül des Zielanalyten zu Stellen des Arrays weniger als 1:5 ist, was zu Stellen mit einzelnen Analytmolekülen führt; und
    (c) Bestimmen des Prozentsatzes von Stellen des Arrays, die ein Zielanalytmolekül enthalten, um die Konzentration des Zielanalyten zu messen.

**2.** Verfahren gemäß Anspruch 1, worin die Moleküle des Zielanalyten optional gelabelte Biomoleküle umfassen.

**3.** Verfahren gemäß Anspruch 2, worin die Biomoleküle aus der Gruppe bestehend aus Proteinen, Nukleinsäuren, Lipiden, Kohlenhydraten, Hormonen, Cytokinen, zellulären Antigenen und Rezeptoren ausgewählt sind.

**4.** Verfahren gemäß Anspruch 3, worin die Biomoleküle Rezeptoren sind, ausgewählt aus der Gruppe bestehend aus neuralen Rezeptoren, Hormonrezeptoren und Nährstoffrezeptoren.

**5.** Verfahren gemäß Anspruch 1, worin der Zielanalyt aus der Gruppe bestehend aus Umweltverunreinigungen, Pestiziden, Insektiziden, Toxinen und Medikamenten ausgewählt ist.

**6.** Verfahren gemäß Anspruch 1, worin das Verhältnis (i) zwischen 1:5 und 1:500 oder (ii) 1:10 ist.

**7.** Verfahren gemäß Anspruch 1, das weiterhin nach dem Schritt des Inkontaktbringens das Versiegeln des Arrays umfasst, so dass die Inhalte einer jeden Stelle nicht aus dieser Stelle entkommen können.

**8.** Verfahren gemäß Anspruch 1, worin das definierte Volumen einer jeden Stelle im Bereich von 30 Femtolitern bis 60 Femtolitern liegt.

**9.** Verfahren gemäß Anspruch 1, worin das Array (i) zwischen 20.000 und 10.000.000 Stellen; oder (ii) zwischen 20.000 und 30.000 Stellen; oder (iii) zwischen 100.000 und 10.000.000 Stellen oder (iv) zwischen 10.000 und 50.000 Stellen umfasst.

**10.** Verfahren gemäß Anspruch 1, worin jedes Stelle des Arrays eine Einfang-Komponente umfasst.

**11.** Verfahren gemäß Anspruch 10, worin die Moleküle des Zielanalyten aus der Gruppe bestehend aus Enzymen, DNA oder Antikörpern ausgewählt sind.

**12.** Verfahren gemäß Anspruch 1, worin das Array eine Mehrzahl von Microwells umfasst, die an einem Ende eines faseroptischen Bündels gebildet sind.

**13.** Verfahren gemäß Anspruch 1, worin das Ermitteln die Detektion einer Änderung in einer optischen Eigenschaft an den besagten Stellen als Indikation der Gegenwart des Zielanalytmoleküls umfasst.

**Revendications**

**1.** Procédé de mesure de la concentration d'un analyte cible dans un échantillon, comprenant le fait :

(a) de fournir un échantillon et une matrice de composants de capture, ledit échantillon comprenant des molécules d'analyte cible, ladite matrice comprenant au moins 1000 sites, chaque site ayant un volume défini entre 10 attolitres et 50 picolitres ;
(b) de mettre en contact ladite matrice avec ledit échantillon afin que des sites contiennent l'analyte cible lié aux composants de capture de sorte que le rapport de molécules d'analyte cible sur des sites de ladite matrice soit inférieur à 1 : 5 ce qui conduit à des sites avec des molécules d'analyte uniques ; et
(c) de déterminer le pourcentage de sites de ladite matrice contenant une molécule d'analyte cible pour mesurer la concentration d'analyte cible.

**2.** Procédé de la revendication 1, dans lequel lesdites molécules d'analyte cible comprennent des biomolécules facultativement marquées.

**3.** Procédé de la revendication 2, dans lequel lesdites biomolécules sont choisies dans le groupe constitué des protéines, des acides nucléiques, des lipides, des hydrates de carbone, des hormones, des cytokines, d'antigènes cellulaires et de récepteurs.

**4.** Procédé de la revendication 3, dans lequel lesdites biomolécules sont des récepteurs choisis dans le groupe constitué de récepteurs neuraux, de récepteurs hormonaux, et de récepteurs de nutriments.

**5.** Procédé de la revendication 1, dans lequel ledit analyte cible est choisi dans le groupe constitué de polluants de l'environnement, de pesticides, d'insecticides, de toxines et de médicaments.

**6.** Procédé de la revendication 1, dans lequel ledit rapport est (i) compris entre 1 : 5 et 1 : 500 ; ou (ii) égal à 1:10.

**7.** Procédé de la revendication 1, comprenant en outre, après l'étape de mise en contact, le scellement de la matrice de sorte que le contenu de chaque site ne puisse pas s'échapper dudit site.

**8.** Procédé de la revendication 1, dans lequel ledit volume défini de chaque site varie de 30 femtolitres à 60 femtolitres.

**9.** Procédé de la revendication 1, dans lequel ladite matrice comprend (i) entre 20000 et 10000000 sites ; ou (ii) entre 20000 et 30000 sites ; ou (iii) entre 100000 et 10000000 sites, ou (iv) entre 10000 et 50000 sites.

**10.** Procédé de la revendication 1, dans lequel chaque site de ladite matrice comprend un composant de capture.

**11.** Procédé de la revendication 10, dans lequel lesdites molécules d'analyte cible sont choisies dans le groupe constitué des enzymes, de l'ADN, ou des anticorps.

**12.** Procédé de la revendication 1, dans lequel la matrice comprend une pluralité de micro-puits formés dans une extrémité d'un faisceau de fibres optiques.

**13.** Procédé de la revendication 1, dans lequel ladite détermination comprend la détection d'un changement d'une propriété optique au niveau desdits sites en tant qu'indication de la présence de ladite molécule d'analyte cible.

FIG. 1

a             b             c             d

silanize      polish      biotinylate

FIG. 2

FIG. 3

(a)                    (b)

FIG. 4

FIG. 5

Digital Readout of Target

$y = 1.0728x + 18.222$
$R^2 = 0.9902$

log (% active wells)

log (moles of target)

FIG. 6

(a)

(b)

**FIG. 7**

(a)        (b)        (c)

FIG. 8

(a)                    (b)                    (c)

FIG. 9

FIG. 10

FIG. 11

(A)

(B)

(C)

Control experiment
without inhibitor:
No delayed
Substrate turnover

FIG. 12

**(A)**

**(B)**

FIG. 13

EP 1 996 717 B1

$k^{off} = 4.6 \times 10^{-3}$ sec

=> Half-life: ln2/ $k^{off}$

= 151 sec = 2 min, 30 sec

**FIG. 14**

EP 1 996 717 B1

80nm particle
~2000 DNA strands

Probe # 1

Probe #2

**FIG. 15**

EP 1 996 717 B1

80nm particle
~2000 DNA strands

Probe #2

Probe # 1

Unlabeled target

EP 1 996 717 B1

**FIG. 16**

**FIG. 17**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9958948 A
- WO 2007044974 A2
- WO 9525116 A
- WO 9535505 A
- US 9809163 W
- US 5700637 A
- US 5807522 A
- US 5445934 A
- US SNS08851203 A
- US 09187289 B
- US 6023540 A
- US 6327410 B
- US 6858394 B
- US 5270163 A
- US 5475096 A
- US 5567588 A
- US 5595877 A
- US 5637459 A
- US 5683867 A
- US 5705337 A
- US 5620850 A
- US 816651 A
- US 7033415 B, Mirkin
- US 7169556 B

- US 6274323 B
- US 20040131322 A, Ye
- WO 2004057386 A
- US 4507466 A
- US 4558120 A
- US 4568737 A
- US 4587329 A
- US 4694064 A
- US 4713975 A
- US 4737550 A
- US 4857599 A
- US 4871779 A
- US 5338532 A
- US 6471968 B
- US 5387617 A
- US 5393797 A
- US 5393795 A
- US 5527524 A
- US 5560929 A
- US 5773527 A
- US 5631329 A
- US 5898005 A
- US 5861319 A
- US 5661025 A

**Non-patent literature cited in the description**

- **SANO, T. ; SMITH, C. L. ; CANTOR, C. R.** *Science,* 1992, vol. 258, 120-122
- **NAM, J. M. ; THAXTON, C. S. ; MIRKIN, C. A.** *Science,* 2003, vol. 301, 1884-1886
- **NIEMEYER, C. M. ; ADLER, M. ; PIGNATARO, B.; ; LENHERT, S. ; GAO, S. ; CHI, L. F. ; FUCHS, H. ; BLOHM, D.** *Nucleic Acids Research,* 1999, vol. 27, 4553-4561
- **ZHOU, H.; ; FISHER, R. J. ; PAPAS, T. S.** *Nucleic Acids Research,* 1993, vol. 21, 6038-6039
- **NIEMEYER, C. M. ; ADLER, M. ; WACKER, R.** *Trends in Biotechnology,* 2005, vol. 23, 208-216
- technical section on cross-linkers. Pierce Chemical. Company catalog, 1994, 155-200
- **HELLER.** *Acc. Chem. Res.,* 1990, vol. 23, 128
- The Handbook - A Guide to Fluorescent Probes and Labeling Technologies
- Clusters and Colloids. VCH, 1994
- Colloidal Gold: Principles, Methods, and Applications. Academic Press, 1991

- **MASSART, R.** *IEEE Taransactions On Magnetics,* 1981, vol. 17, 1247
- **AHMADI, T. S. et al.** *Science,* 1996, vol. 272, 1924
- **HENGLEIN, A. et al.** *J. Phys. Chem.,* 1995, vol. 99, 14129
- **CURTIS, A. C. et al.** *Angew. Chem. Int. Ed. Engl.,* 1988, vol. 27, 1530
- **WELLER.** *Angew. Chem. Int. Ed. Engl.,* 1993, vol. 32, 41
- **HENGLEIN.** *Top. Curr. Chem.,* 1988, vol. 143, 113
- **HENGLEIN.** *Chem. Rev.,* 1989, vol. 89, 1861
- **BRUS.** *Appl. Phys. A.,* 1991, vol. 53, 465
- **BAHNCMANN.** Photochemical Conversion and Storage of Solar Energy. 1991, 251
- **WANG ; HERRON.** *J. Phys. Chem.,* 1991, vol. 95, 525
- **OLSHAVSKY et al.** *J. Am. Chem. Soc.,* 1990, vol. 112, 9438

- **USHIDA et al.** *J. Phys. Chem.,* 1992, vol. 95, 5382
- **CHAN ; NIE.** *Science,* 1998, vol. 281, 2016
- **SOOKLAL.** *Adv. Mater.,* 1998, vol. 10, 1083
- **THOMAS et al.** *Biophysical Journal,* 2004, vol. 86 (6), 3959
- **TOMALIA.** *Advanced Materials,* 1994, vol. 6, 529
- **ANGEW.** *Chem. Int. Ed. Engl.,* 1990, vol. 29, 138
- **TOMALIA et al.** *Chem. Int. Ed. Engl.,* 1990, vol. 29, 5305
- **YIN et al.** *J. Am. Chem. Soc.,* 1998, vol. 120, 2678
- **SINGH et al.** *Clin. Chem.,* 1994, vol. 40, 1845
- **BAKER et al.** *Anal. Chem.,* 1997, vol. 69, 990
- **URDEA ; HORN.** *Science,* 1993, vol. 261, 534
- *Analytical Chemistry,* 2000, vol. 72, 5535-5541
- *Science,* 2002, vol. 296, 1836-1838
- *Science,* 2002, vol. 295, 1503-1506
- *J. Am. Chem. Soc.,* 2004, vol. 126, 5932-5933
- **FRENS.** *Nature Phys. Sci.,* 1973, vol. 241, 20
- **GRABAR.** *Anal Chem.,* 1995, vol. 67, 735
- **NIEMEYER, C. M. ; ADLER, M. ; PIGNATARO, B. ; LENHERT, S. ; GAO, S. ; CHI, L. F. ; FUCHS, H. ; BLOHM, D.** *Nucleic Acids Research,* 1999, vol. 27, 4553-4561
- **ZHOU, H. ; FISHER, R J. ; PAPAS, T. S.** *Nucleic Acids Research,* 1993, vol. 21, 6038-6039
- **NIEMEYER, C. M.; ; ADLER, M. ; WACKER, R.** *Trends in Biotechnology,* 2005, vol. 23, 208-216
- **WHITESIDES, G. M.** *Nature Biotechnology,* 2003, vol. 21, 1161-1165
- **RONDELEZ, Y. ; TRESSET, G. ; TABATA, K. V. ; ARATA, H. ; FUJITA, H. ; TAKEUCHI, S. ; NOJI, H.** *Nature Biotechnology,* 2005, vol. 23, 361-365
- **NAKANO, M. ; KOMATSU, J. ; MATSUURA, S. ; TAKASHIMA, K. ; KATSURA, S. ; MIZUNO, A.** *Journal of Biotechnology,* 2003, vol. 102, 117-124
- **NAGAI, H. ; MURAKAMI, Y. ; YOKOYAMA, K. ; TAMIYA, E.** *Biosensors and Bioelectronics,* 2001, vol. 16, 1015-1019
- **LIPMAN, A. E. ; SHULER, B. ; BAKAJIN, O. ; EATON, W. A.** *Science,* 2003, vol. 301, 1233-1235
- **CHIU, D. T. ; WILSON, C. F. ; RYTTSEN, F.; ; STROMBERG, A. ; FARRE, C. ; KARLSSON, A. ; NORDHOLM, S. ; GRAGGAR, A. ; MODI, B. P.; ; MOSCHO, A.** *Science,* 1999, vol. 283, 1892-1895
- **RISSIN, D. M. ; WALT, D. R.** *Journal of the American Chemical Society*
- **PANTANO, P. ; WALT, D. R.** *Chemistry of Materials,* 1996, vol. 8, 2832-2835
- **MONK, D. J. ; UEBERFELD, J. ; WALT, D. R.** *Journal of Materials Chemistry,* 2005, vol. 15, 4361-4366
- **SONG, L. N. ; AHN, S. ; WALT, D. R.** *Emerging Infectious Diseases,* 2005, vol. 11, 1629-1632
- **LEE, J. Y. ; LI, H. W. ; YEUNG, E. S.** *Journal of Chromatography A,* 2004, vol. 1053, 173-179
- **XUE, Q. F. ; YEUNG, E. S.** *Nature,* 1995, vol. 373, 681-683
- **FOQUET, M. ; KORLACH, J. ; ZIPFEL, W. R. ; WEBB, W. W. ; CRAIGHEAD, H. G.** *Analytical Chemistry,* 2004, vol. 76, 1618-1626
- **GRATZL, M. ; LU, H. ; MATSIMOTO, T. ; YI, C. ; BRIGHT, G. R.** *Analytical Chemistry,* 1999, vol. 71, 2751-2756
- **STAMOU, D. ; DUSCHL, C. ; DELAMARCHE, B. ; VOGEL, H.** *Angewandte Chemie-International Edition,* 2003, vol. 42, 5580-5583
- **GOSALIA, D. N. ; DIAMOND, S. L.** *Proceeding of the National Academy of Sciences USA,* 2003, vol. 100, 8721-8726
- **LU, H. P. ; XUN, L. Y. ; XIE, X. S.** *Science,* 1998, vol. 282, 1877-1882
- **TAYLOR, J. R.** An Introduction to Error Analysis. University Science Books, 1997
- **WHEELER, A. R. ; THRONDSET, W. R. ; WHELAN, R. J. ; LEACH, A. M. ; ZARE, R. N. ; LIAO, Y. H. ; FARRELL, K. ; MANGER, L D. ; DARIDON, A.** *Analytical Chemistry,* 2003, vol. 75, 3581-3586
- **ANGENENDT P. ; LEHRACH H. ; KREUTZBERGER J. ; GLÖKLER J.** *Proteomics,* 2005, vol. 5, 420-425